(19) Europäisches Patentamt
European Patent Office
Office européen des brevets

(11) Veröffentlichungsnummer: **0 242 686 B1**

# (12) EUROPÄISCHE PATENTSCHRIFT

(45) Veröffentlichungstag der Patentschrift: **29.01.92**

(51) Int. Cl.5: **C07D 513/04**, C07D 498/04, //A61K31/425,(C07D513/04, 277:00,235:00),(C07D498/04, 263:00,235:00)

(21) Anmeldenummer: **87105054.8**

(22) Anmeldetag: **06.04.87**

## (54) Verfahren zur Herstellung von D-(+)-Biotin.

(30) Priorität: **19.04.86 DE 3613245**
**07.02.87 DE 3703872**

(43) Veröffentlichungstag der Anmeldung:
**28.10.87 Patentblatt 87/44**

(45) Bekanntmachung des Hinweises auf die Patenterteilung:
**29.01.92 Patentblatt 92/05**

(84) Benannte Vertragsstaaten:
**CH DE FR GB IT LI**

(56) Entgegenhaltungen:
**EP-A- 0 094 776**
**DE-A- 2 058 234**
**US-A- 4 009 172**

**JOURNAL OF THE AMERICAN CHEMICAL SOCIETY, Band 97, 1975, Seiten 5936-5938; P.N.Confalone et al.**

**CHEMICAL ABSTRACTS OF JAPAN, Band 99, 1983, Seite 622, Spalte 1, Zusammenfassungsnr. 70655k; I.Lalezari et al.**

**JOURNAL AMERICAN CHEMICAL SOCIETY, Band 105, 1983, Seiten 5946-5948; R.A.Volkmann et al.**

**CHIMIA 41, Nr.5, Mai 1987, E.Poetsch et al.**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Poetsch, Eike, Dr.**
**Am Buchwald 4**
**W-6109 Mühltal 6(DE)**
Erfinder: **Casutt, Michael, Dr.**
**Adolf-Kolping-Strasse 19**
**W-6102 Pfungstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 242 686 B1

**Beschreibung**

Die Erfindung betrifft ein Verfahren zur Herstellung von D-(+)-Biotin aus L-Cystein oder L-Cystin oder L-Serin über ein optisch aktives (7R)-1H,3H-Imidazo[1,5-c]azol als Zwischenprodukt.

Der Erfindung lag die Aufgabe zugrunde, ein neues Verfahren für die Herstellung optisch aktiven D-(+)-Biotins bereitzustellen, welches die Durchführung einer Racematspaltung und damit das Verwerfen oder Rückführen des unerwünschten Enantiomeren vermeidet.

Verfahren zur stereospezifischen Synthese von D-(+)-Biotin aus Zuckern geeigneter Konfiguration sind bekannt. So wird in Tetrahedron Letters Nr. 32, S. 2765-2766 (1975), als Ausgangsmaterial D-Mannose, in Agric. Biol. Chem. Nr. 42, S. 465 (1978), D-Glucose und in den DE-OS 31 22 562 und DE-OS 33 20 140 D-Arabinose als chirales Ausgangsmaterial verwendet.

Alle diese Verfahren sind jedoch durch eine hohe Zahl von Syntheseschritten mit folglich geringer Gesamtausbeute gekennzeichnet. Die aufgrund ihrer Zuckernatur meist nicht kristallisierbaren Zwischenstufen werden oft nur in unbefriedigender Reinheit erhalten und erfordern, bedingt durch ihre Polyfunktionalität und der damit verbundenen chemischen Labilität die Einhaltung vergleichsweise enger Reaktionsparameter. Eine Reihe von Zuckern ist auch aus natürlichen Quellen nicht zugänglich, was einen hohen Preis zur Folge hat.

Die Verwendung von L-Cystein, wie aus US-4009172, US-4130713, US-4337345 und Journal of the American Chemical Society Nr. 99, S. 7020 (1977) bekannt, vermeidet zwar die Handhabung labiler Zwischenstufen, führt hingegen über insgesamt 18 Reaktionsstufen unter Abtrennung unerwünschter Isomere nur in unbefriedigender Ausbeute zu optisch aktivem D-(+)-Biotin.

In einem weiteren Verfahren werden in Journal of the American Chemical Society Nr. 105, S. 5946 (1983) und in der EP-OS 0 094776 substituierte 3H,5H-Imidazo[1,5-c]tetrahydrothiazole beschrieben, aus denen nach Racematspaltung optisch aktives Biotin erhalten wird.

Da die vergleichsweise hohe Stufenzahl verbunden mit teilweise mäßigen Ausbeuten und der Notwendigkeit einer optischen Trennung,auch diese Ausgangsstoffe als zur Herstellung von D-(+)-Biotin wenig geeignet erscheinen lassen, bestand weiterhin Bedarf an einem geeigneten Verfahren zur einfachen, ökonomischen und stereospezifischen Herstellung von D-(+)-Biotin.

Es wurde nun überraschend gefunden, daß D-(+)-Biotin aus den natürlich vorkommenden Aminosäuren L-Cystein oder L-Cystin oder L-Serin über ein optisch aktives (7R)-1H,3H-Imidazo[1,5-c]azol der Formel I als Zwischenprodukt auf stereospezifische Weise ohne zusätzliche Racemattrennung hergestellt werden kann. Gegenstand der Erfindung ist daher ein Verfahren zur Herstellung von D-(+)-Biotin aus L-Cystein oder L-Cystin oder L-Serin, dadurch gekennzeichnet, daß die Synthese über ein aus der Verbindung der Formel I

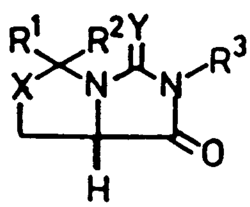

I

hergestelltes Zwischenprodukt der Formel XI geführt wird,

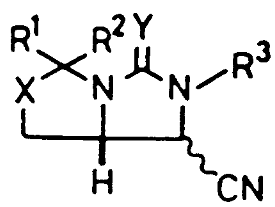

XI

worin jeweils

$R^1$ und $R^2$ jeweils unabhängig voneinander H, unsubstituiertes oder substituiertes niederes Alkyl, Cycloalkyl, Aryl, Aralkyl oder Heteroaryl oder zusammengenommen unsubstituiertes oder

substituiertes Alkylen oder Heteroalkylen,

$R^3$ H oder eine für ein Stickstoffatom geeignete Schutzgruppe und

X und Y jeweils unabhängig voneinander O oder S bedeuten.

In dieser Formel bedeutet X vorzugsweise S und Y vorzugsweise O, insbesonder sind gleichzeitig X = S und Y = O. Die Reste $R^1$ und $R^2$ bedeuten vorzugsweise H, $C_1$-$C_4$-Alkyl, unsubstituiertes oder durch $C_1$-$C_3$ Alkyl und/oder Alkoxy ein- oder mehrfach substituiertes Phenyl oder Benzyl, insbesonder sind gleichzeitig $R^1$ = H, $R^2$ = Phenyl. Vorzugsweise bedeutet der Rest $R^3$ unsubstituiertes oder durch eine oder mehrere, insbesonders bevorzugt eine oder zwei, $C_1$-$C_4$ Alkyl- und/oder $C_1$-$C_4$-Alkoxygruppen substituiertes Benzyl, insbesondere unsubstituiertes Benzyl, daneben auch $C_3$-$C_5$ Alk-2-enyl oder $C_3$-$C_6$ Trialkylsilyl. Bei mehrfacher, vorzugsweise zweifacher Substitution eines Phenylrings sind die Substituenten vorzugsweise gleich, sie können aber auch verschieden sein. Sie befinden sich vorzugsweise in 4- und/oder 2-Stellung, sie können aber auch in 3-, 5- und/oder 6-Stellung stehen.

Weiterhin ist Gegenstand der vorliegenden Erfindung ein Verfahren zur Herstellung von D-(+)-Biotin aus Verbindungen der Formel I, indem

a) I mit komplexen Hydriden Zu einem Alkohol der Formel IX reduziert wird,

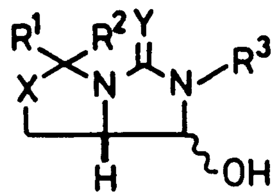

IX

worin $R^1$, $R^2$, $R^3$, X und Y die angegebene Bedeutung besitzen, der in einen aktivierten Ester der Formel X überführt wird,

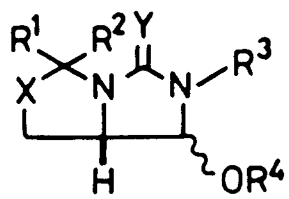

X

worin $R^1$ $R^2$ $R^3$ X und Y die angegebene Bedeutung besitzen und der Rest $R^4$ für eine aktivierende Estergruppe steht, der mit einem Alkali- oder Erdalkalicyanid oder einem Cyanosilan zu einem Nitril der Formel XI umgesetzt wird,

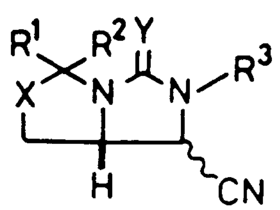

XI

worin $R^1$, $R^2$, $R^3$, X und Y die angegebene Bedeutung besitzen.

Weiterhin ist Gegenstand der vorliegenden Erfindung ein Verfahren zur Herstellung von D-(+)-Biotin, wobei das Nitril der Formel XI mit einer Base oder einer Säure zu einem Säurederivat der Formel XII umgesetzt wird,

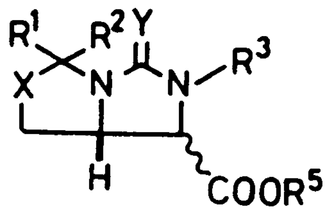

XII

worin $R^1$, $R^2$, $R^3$, X und Y die angegebene Bedeutung besitzen und $R^5$ H oder niederes Alkyl bedeutet, das unter Wasserabspaltung zu einem Lacton der Formel XIII cyclisiert wird,

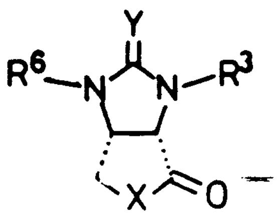

XIII

worin $R^3$, X und Y die angegebene Bedeutung besitzen und $R^6$ H oder $R^1R^2CH$ bedeutet, das nach bekannten Verfahren in D-( + )-Biotin überführt wird,

b) Weiterhin ist Gegenstand der vorliegenden Erfindung ein Verfahren zur Herstellung von D-( + )-Biotin, wobei das Nitril der Formel XI zu einer Oxoverbindung der Formel XIV umgesetzt wird,

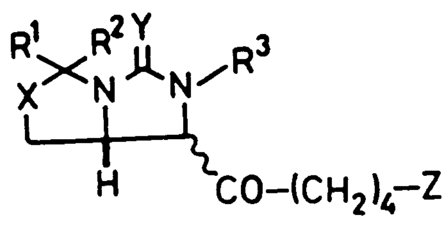

XIV

worin $R^1$, $R^2$, $R^3$, X und Y die angegebene Bedeutung besitzen und Z $OR^5$ oder $COOR^5$ bedeutet, wobei $R^5$ H, niederes Alkyl, Cycloalkyl oder Aryl bedeutet, die durch Behandeln mit einer Säure und/oder einem Reduktionsmittel zu einem Imidazolidin der Formel XV gespalten wird,

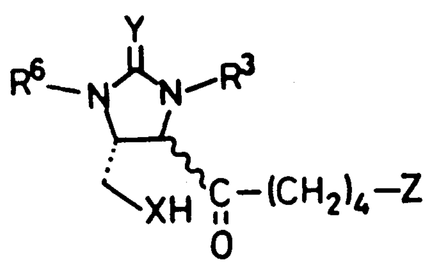

XV

worin $R^3$, $R^6$, X, Y und Z die angegebene Bedeutung besitzen, das unter Einwirkung einer Base zu einem Biotinderivat der Formel XVI cyclisiert wird,

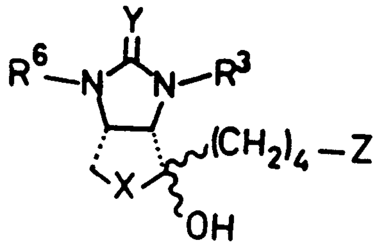

XVI

worin $R^3$, $R^6$, X, Y und Z die angegebene Bedeutung besitzen, das nach bekannten Verfahren in D-(+)-Biotin überführt wird. Weiterhin ist Gegenstand der vorliegenden Erfindung ein Verfahren zur Herstellung von D-(+)-Biotin, wobei das Nitril der Formel XI mit einem Reduktionsmittel zu einem Aldehyd der Formel XVII umgesetzt wird,

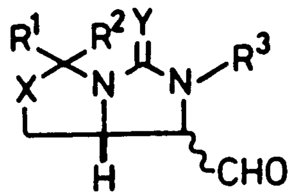

XVII

worin $R^1$, $R^2$ $R^3$, X und Y die angegebene Bedeutung besitzen, der mit einer Organophosphorverbindung zu einer ungesättigten Carbonsäure der Formel XVIII kondensiert wird,

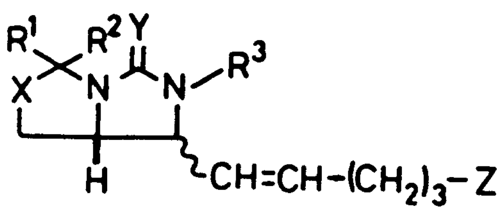

XVIII

worin $R^1$, $R^2$, $R^3$, X, Y und Z die angegebene Bedeutung besitzen, die durch eine Säure und/oder ein Reduktionsmittel zu einem Biotinderivat der Formel XIX umgesetzt wird,

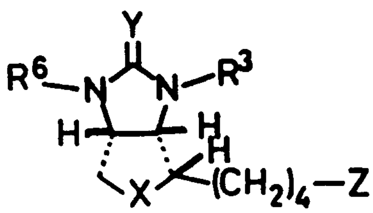

XIX

worin $R^3$, $R^6$, X, Y und Z die angegebene Bedeutung besitzen, das nach bekannten Verfahren in D-(+)-Biotin überführt wird,

d) Weiterhin ist Gegenstand der vorliegenden Erfindung ein Verfahren zur Herstellung von D-(+)-Biotin, wobei das Nitril der Formel XI in eine Oxoverbindung der voranstehend angegebenen Formel XIV überführt wird, und diese unter Säureeinwirkung zu einem Biotinderivat der Formel XX umgesetzt wird,

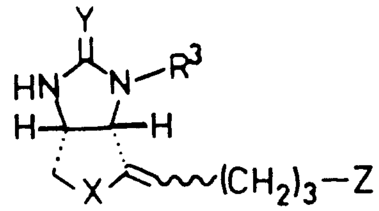

XX

worin $R^3$, X, Y und Z die angegebene Bedeutung besitzen, das nach bekannten Verfahren in D-(+)-Biotin überführt wird.

Weiterhin sind Verbindungen der Formel XI Gegenstand der Erfindung, da sie wertvolle Zwischen- und Ausgangsprodukte einer Vielzahl von Synthesemöglichkeiten von optisch aktivem D-(+)-Biotin darstellen.

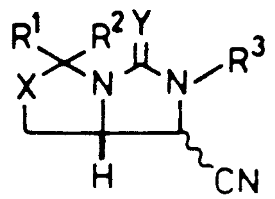

XI

Hierbei besitzen $R^1$, $R^2$, X und Y die angegebene Bedeutung und $R^3$ stellt eine für ein Stickstoffatom geeignete Schutzgruppe dar.

Bevorzugt sind Verbindungen der Formel XI, worin X Schwefel und Y Sauerstoff bedeuten. Insbesondere bevorzugt ist die Verbindung XIa, worin $R^1$ Wasserstoff, $R^2$ Phenyl und $R^3$ Benzyl bedeuten.

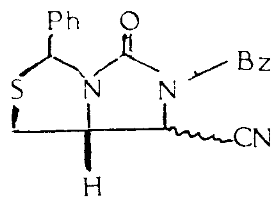

XIa

Die Herstellung der Verbindungen der Formel I erfolgt nach an sich bekannten Methoden, wie sie in der Literatur (z. B. in Standardwerken wie Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formel II sind bekannt oder können nach bekannten Methoden aus L-Cystein oder L-Serin hergestellt werden, wie beispielsweise in Schöberl, Hamm, Chem. Ber. 81 [1948], 210 und Karabinos, Szabo, J. Amer. Chem. Soc. 66 [1944], 649 angegeben, indem man die freien Aminosäuren oder deren Säureadditionssalze mit einem Alkali- oder Erdalkalimetallcyanat oder -thiocyanat in einem geeigneten Lösungsmittel wie Wasser, Alkoholen oder deren Gemischen, vorzugsweise bei erhöhter Temperatur, miteinander umsetzt und das erhaltene Zwischenprodukt in situ unter Einwirkung einer Säure, beispielsweise einer Mineralsäure, cyclisiert.

Die Umsetzung der Hydantoine der Formel II mit Carbonylverbindungen der Formel III zu den Bicyclen der Formel IV kann nach den für Acetalisierungen bekannten und gebräuchlichen Verfahren erfolgen wie sie beispielsweise in Houben-Weyl, Methoden der Organischen Chemie, Bd. VI/3, S. 199, beschrieben sind. Vorzugsweise erfolgt die Umsetzung der Reaktionspartner unter Zusatz eines wasserentziehenden Mittels wie z. B. einer Säure wie Schwefelsäure, Phosphorsäure, Chlorwasserstoff, p-Toluolsulfonsäure, einem Säurederivat wie Phosphorpentoxid, Phosphortrichlorid, Phosphorpentachlorid, Phosphoroxychlorid, einem Metallsalz wie wasserfreiem Calciumchlorid, Kupfersulfat, Eisen(III)-chlorid, einem sauren Ionenaustauscher oder Molekularsieben. Die Entfernung des gebildeten Reaktionswassers kann auch durch azeotrope Destillation mit einem geeigneten Lösungsmittel wie Benzol, Toluol, Chloroform, Dichlormethan erfolgen. Schließlich ist es auch möglich, anstelle einer freien Carbonylverbindung der Formel III, deren Acetal mit einem geeigneten Alkohol, vorzugsweise einem niederen Alkohol, zur Herstellung der Verbindungen der Formel IV zu verwenden. Zweckmäßigerweise wird der bei der Reaktion freiwerdende Alkohol laufend aus dem

Reaktionsgemisch entfernt, beispielsweise durch Destillation oder Adsorption. Auch kann durch einen Überschuß an Acetal von Oxoverbindung III gebildetes Reaktionswasser entfernt werden.

Für die Umsetzung mit den Hydantoinen der Formel II verwendet man vorteilhaft ein Äquivalent an Carbonylverbindung III bzw. deren Acetal, welche gleichzeitig als Lösungsmittel dienen können. Zweckmäßiger ist es jedoch, ein zusätzliches inertes Lösungsmittel zuzusetzen. Als inerte Lösungsmittel eignen sich vorzugsweise Kohlenwasserstoffe wie Pentan, Hexan, Cyclohexan, Benzol, Toluol oder Xylol sowie chlorierte Kohlenwasserstoffe wie Dichlormethan, Chloroform oder Tetrachlorkohlenstoff.

Das Stickstoffatom in den bicyclischen Verbindungen der Formeln I, VIII, IX, X, XI, XII, XIII, XIV, XV, XVI, XVII, XVIII, XIX und XX wird durch Schutzgruppe $R^3$ geschützt, worin $R^3$ z.B. Benzyl, 4-Methoxybenzyl, 3,4-Dimethoxybenzyl, 4-Methylbenzyl, Allyl, Methallyl, Crotyl, Methoxymethyl, Trimethylsilyl, tert-Butyldimethylsilyl oder tert-Butyldiphenylsilyl bedeutet. Diese Schutzgruppen werden nach bekannten Verfahren, wie sie beispielsweise aus Mac Omie, Protective Groups in Organic Chemistry, Plenum Press, New York, 1973 entnommen werden können, durch Umsetzen der entsprechenden reaktiven Halogenide mit den Bicyclen der Formel IV, unter Erhalt von (7R)-1H,3H-Imidazo[1,5c]azolen der Formel I, eingeführt.

Vorzugsweise erfolgt die Umsetzung der Reaktionspartner in einem geeigneten Lösungsmittel unter Zusatz eines basischen Reagenzes. Gut geeignete Lösungsmittel sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan.

Eine bevorzugte Reaktionsweise ist die Umsetzung einer bicyclischen Verbindung der Formel IV mit einer reaktiven, den Rest $R^3$ tragenden Verbindung, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid, Alkalimetallhydride wie Natriumhydrid, Amide wie Natriumamid, Lithiumdiisopropylamid, Alkoholate wie Natriummethylat, Natriumethylat, Lithiumethylat oder organische Basen wie Triethylamin, Pyridin, Lutidin, Collidin, Imidazol, 4-(N,N-Dimethylamino)-pyridin oder Chinolin von Bedeutung sind.

Die Reaktionstemperatur liegt gewöhnlich zwischen -50 °C und +250 °C, vorzugsweise zwischen -20 °C und +80 °C. Bei diesen Temperaturen sind die Reaktionen in der Regel nach 15 Minuten bis 48 Stunden beendet.

In einem weiteren Verfahren zur Herstellung der Verbindungen der Formel II setzt man L-Cystein oder L-Serin mit einer Carbonylverbindung der Formel III zu einem Azolidin V um. Verbindungen der Formel V und Verfahren zu deren Herstellung sind beispielsweise bekannt aus Schubert, J. Biol. Chem. 114 (1936), 341, Uskovič et al., J. Amer. Chem. Soc. 97 (1975), 5936, Lieberman et al., ibid. 70 (1948), 3094 und US-3957794 sowie US-4009172. Ebenfalls geeignet sind die zur Herstellung der Verbindungen der Formel IV angegebenen Reaktionsbedingungen.

Azolidine der Formel V können mittels Alkalimetall- oder Erdalkalimetallcyanaten oder -thiocyanaten unter den bereits für die Herstellung von Verbindungen der Formel II angegebenen Reaktionsbedingungen in Verbindungen der Formel IV und weiter wie angegeben in die Imidazo[1,5-c]azole der Formel I überführt werden.

Zweckmäßiger ist es jedoch, die Azole der Formel V mit einem Organoisocyanat oder -isothiocyanat der Formel VI direkt zu den Imidazo[1,5-c]azolen der Formel I umzusetzen. Organoisocyanate und -isothiocyanate der Formel VI sind bekannt oder können nach bekannten Methoden wie beispielsweise in Houben-Weyl, Methoden der Organischen Chemie, Bde. VIII, S. 75 u. IX, S. 773, beschrieben, erhalten werden.

Aus Lieberman, J. Amer. Chem. Soc. 70 (1948), 3094 und Crabb et al., Tetrahedron 29 (1973), 3389, ist die Umsetzung von Hydantoinen mit Phenyl- und Methylisocyanat bekannt. Nach diesen Methoden kann auch die Reaktion der Azole der Formel V mit den Organoisocyanaten oder -isothiocyanaten der Formel VI erfolgen. Vorzugsweise wird die Umsetzung der Reaktionspartner in einem geeigneten Lösungsmittel durchgeführt wie z. B. Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Geeignet sind auch basische Lösungsmittel wie Pyridin, Lutidin, Collidin, Diethylamin oder Triethylamin sowie Gemische dieser Basen mit den voranstehend angegebenen Lösungsmitteln.

Gegebenenfalls kann es zweckmäßig sein, die primär gebildete Carbamoyl- oder Thiocarbamoylverbindung zu isolieren und in einem separaten Reaktionsschritt unter Wasserabspaltung zu cyclisieren. Als

wasserabspaltende Mittel eignen sich beispielsweise Säuren wie Schwefelsäure, Chlorwasserstoff, Toluolsulfonsäure oder Basen wie Natrium- oder Kaliumhydroxid. Man kann dabei in Gegenwart oder Abwesenheit eines inerten Lösungsmittels bei Temperaturen zwischen etwa 0 °C und 150 °C, vorzugsweise zwischen etwa 20 C° und 100 °C, arbeiten; als Lösungsmittel kommen z. B. Wasser und Alkohole wie Methanol, Ethanol, Isopropanol oder Butanol in Betracht.

In einem weiteren Verfahren zur Herstellung der Verbindungen der Formel II setzt man L-Cystin mit einem Alkalimetall- oder Erdalkalimetallcyanat oder -thiocyanat zu einem Bishydantoin der Formel VII um. Dazu kann von den gleichen Methoden Gebrauch gemacht werden, die bereits zur Herstellung der Hydantoine der Formel II angegeben wurden, wobei gleiche oder ähnliche Reaktionsbedingungen zur Anwendung gelangen.

Aus den Bishydantoinen der Formel VII lassen sich durch Behandeln mit einem Reduktionsmittel Hydantoine der Formel II (X = S) erhalten, aus denen nach den voranstehend angegebenen Methoden die Imidazo[1,5-c]thiazole der Formel I (X = S) hergestellt werden. Geeignete Reduktionsmittel sind beispielsweise Houben-Weyl, Methoden der Organischen Chemie, Bd. 15/1, S. 798, zu entnehmen. Vorzugsweise eignen sich Natrium/flüssiger Ammoniak, Zink/Säure oder Phosphoniumiodid zur reduktiven Spaltung der Disulfid-Bindung. Zweckmäßigerweise führt man die Reduktion der Bishydantoine der Formel VII mit einem Äquivalent, insbesondere mit einem Überschuß Reduktionsmittel durch in einem geeigneten, der chemischen Natur des Reagenzes angepaßten Lösungsmittel wie z. B. Wasser, flüssigem Ammoniak, Alkoholen wie Methanol, Ethanol, Isopropanol, Säuren wie Salzsäure, Schwefelsäure, Ameisensäure, Essigsäure, Ethern wie Diethylether, THF oder Dioxan oder auch deren Gemischen, zweckmäßig bei Temperaturen zwischen etwa -50 °C und +150 °C.

Ein besonders vorteilhaftes Verfahren zur Spaltung der Disulfidbindung in den Verbindungen der Formel VII besteht in deren Thiolyse mit einem geeigneten Mercaptan wie z. B. Thiophenol, Butan-1,4-dithiol oder 1,4-Dithio-threitol analog zu der bei Hase, Walter, Inst. J. Pept. Prot. Res. 5 (1973), 283, angegebenen Arbeitsweise. Die Umsetzung der Reaktionspartner erfolgt zweckmäßigerweise in einem geeigneten Lösungsmittel wie z. B. wäßrigen Alkalimetallhydroxidlösungen, Chlorkohlenwasserstoffen oder flüssigem Ammoniak bei Temperaturen zwischen etwa -40 °C und +120 °C.

Weiterhin können die Bishydantoine der Formel VII mit einer Schutzgruppe der Formel $R^3$ versehen werden. Zur Herstellung dieser geschützten Bishydantoine der Formel VIII kann von den bereits für die Herstellung der Verbindungen der Formel I aus den Bicyclen der Formel IV angegebenen Methoden Gebrauch gemacht werden, wobei gleiche oder ähnliche Reaktionsbedingungen zur Anwendung gelangen.

Ein weiteres Verfahren zur Herstellung der geschützten Bishydantoine der Formel VIII besteht in der Umsetzung von L-Cystin mit einem Organoisocyanat oder -isothiocyanat der angegebenen Formel VI in Analogie zur Herstellung von Verbindungen der Formel I aus Azolidinen der Formel V, wobei man sich gleicher oder ähnlicher Verfahren bedient, wie bereits für die Herstellung der Verbindungen der Formel I angegeben.

Zur Überführung der Verbindungen der Formel VIII in die (7aR)-1H,3H-Imidazo[1,5-c]thiazole der Formel I (X = S) werden diese in einer der Herstellung der Hydantoine der Formel II aus den Disulfiden der Formel VII entsprechenden Weise unter Anwendung gleicher oder ähnlicher Verfahren und Reaktionsbedingungen mit einem Reduktionsmittel oder einem die Thiolyse bewirkenden Reagenz behandelt und darauf, entsprechend der Herstellung von Verbindungen der Formel V, mit einer Carbonylverbindung der Formel III umgesetzt.

Ein Verfahren zur Herstellung von D-(+)-Biotin aus I besteht darin, daß eine Oxoverbindung der Formel I mit komplexen Hydriden zu einem Alkohol der Formel IX reduziert wird. Geeignete Verfahren sind beispielsweise Houben-Weyl, Methoden der Organischen Chemie, Bd. 4/1c + 4/1d, zu entnehmen.

Geeignete Komplexe Hydride sind beispielsweise Borane, wie Diboran, Natiumboranat, Lithium-cyanotrihydroborat, Metallhydride wie Natriumhydrid, Aluminiumhydrid, Siliciumhydride wie Triethylsilan, Tributylzinnhydrid und gemischte Hydride wie Lithiumalanat, Natriumalanat, Natrium-bis(2-methoxy-ethoxy)-dihydroaluminat, Kaliumboranat oder Lithiumboranat.

Die Umsetzung der Oxoverbindungen der Formel I mit den Reduktionsmitteln erfolgt zweckmäßig in einem geeigneten Lösungsmittel bei Temperaturen zwischen etwa -100 °C und +150°, insbesondere zwischen etwa 0° und 100°; als Lösungsmittel kommen je nach der chemischen Natur des Reduktionsmittel z.B. Wasser, Alkohole wie Methanol, Ethanol, Isopropanol, Butanol, Ether wie Tetrahydrofuran, Dioxan, Diethylether, Ethylenglykoldimethylether und Kohlenwasserstoffe wie Pentan, Cyclohexan, Benzol, Toluol in Betracht.

Zur Aktivierung der Hydroxylgruppe in den Alkoholen der Formel IX werden diese mit einer reaktiven, den Rest $R^4$ tragenden Verbindung $R^4$-Q umgesetzt, worin $R^4$ beispielsweise Alkanoyl wie Acetyl, Aroyl wie Benzoyl, 4-Nitrobenzoyl, Alkylsulfonyl wie Methansulfonyl, Ethansulfonyl, Trifluormethansulfonyl, Arylsulfonyl wie Phenylsulfonyl, Aralkylsulfonyl wie Benzylsulfonyl bedeutet. Weiterhin ist $R^4$ ein Rest Azol-M, worin Azol für einen stickstoffhaltigen, unsubstituierten oder substituierten und/oder kondensierten Fünfring steht wie beispielsweise Imidazol, 1,2,4-Triazol, 1,2,3-Triazol, Benzotriazol, Benzimidazol, Pyrazol, 3,5-Dimethylpyrazol, Indazol, und M CO, CS, SO, $SO_2$, S bedeutet. Vorzugsweise bedeutet $R^4$ Acetyl, Imidazol-1-ylcarbonyl, Imidazol-1-ylsulfinyl, Imidazol-1-ylsulfonyl. Q bedeutet Halogen, Alkoxy, Alkanoyloxy oder ein weiterer Rest Azol.

Somit sind zur Herstellung von aktiven Estern der Formel X insbesondere die Umsetzung von Acetylchlorid, Acetanhydrid, 1,1'-Carbonyldiimidazol, 1,1'-Sulfinyldiimidazol und 1,1'-Sulfonyldiimidazol mit Alkoholen der Formel IX geeignet.

Eine bevorzugte Reaktionsweise ist die Umsetzung eines Alkohols der Formel IX mit einer reaktiven, den Rest $R^4$ tragenden Verbindung, vorzugsweise in einem basischen Milieu, wobei als Basen insbesondere organische Basen wie Triethylamin, Pyridin, Lutidin, Collidin oder Chinolin geeignet sind. Die Reaktionstemperatur liegt gewöhnlich zwischen -50 °C und +150 °C, vorzugsweise zwischen -20 °C und +80 °C. Bei diesen Temperaturen sind die Umsetzungen in der Regel nach 15 Minuten bis 48 Stunden beendet.

Zur Überführung in Nitrile der Formel XI können die aktivierten Ester der Formel X mit einem Cyanid umgesetzt werden, zweckmäßig mit einem Metallcyanid wie Natriumcyanid, Kaliumcyanid oder Kupfercyanid oder einem Cyanosilan wie z.B. Trimethylsilylcyanid, $\beta$-Trimethylsilylpropionitril oder Diethylaluminiumcyanid, z.B. in einem inerten Lösungsmittel wie Dichlormethan, Toluol, Pyridin, Dimethylformamid, Dimethylsulfoxid, N-Methylpyrrolidon oder Hexamethylphosphorsäuretriamid bei Temperaturen zwischen -50 °C und 200 °C.

Ein bevorzugtes Verfahren zur Herstellung von Nitrilen der Formel XI aus aktivierten Estern der Formel X, worin $R^4$ ein Rest Azol-M mit der voranstehend angegebenen Bedeutung ist, besteht in einer zusätzlichen Aktivierung der Ester der Formel X durch deren N-Alkylierung. Hierfür geeignete Alkylierungsmittel sind beispielsweise Alkyl-, Alkenyl- und Aryliodide-, -bromide, -chloride, -sulfate und -sulfonate. Deren Umsetzung mit den aktivierten Estern der Formel X erfolgt zweckmäßigerweise in einem geeigneten Lösungsmittel wie beispielsweise Ethern wie Diethylether, Tetrahydrofuran, Dioxan oder Ketonen wie Aceton, Diethylketon, Methylisobutylketon; sie kann jedoch auch ohne Lösungsmittelzusatz erfolgen.

Ein weiteres bevorzugtes Verfahren zur Herstellung von Nitrilen der Formel XI aus Estern der Formel X, besteht in der Umsetzung letzterer mit einem Cyanosilan nach Methoden wie sie beispielsweise M.T. Reetz et al., Tetrahedron 39 (1983) 961; T. Hiyama et al., Synthesis 1986 689 oder Houben-Weyl, Bd. E5, S. 1389 ff (1985) zu entnehmen sind.

Zu dieser Reaktion besonders geeignet sind die Verbindungen der Formel X, worin $R^4$ Alkanoyl oder Aroyl, insbesondere Alkanoyl wie Acetyl, bedeutet. Trimethylsilylcyanid erweist sich dabei als ein besonders geeignetes Cyanierungsreagenz. Zweckmäßigerweise setzt man die Reaktionspartner in einem inerten Lösungsmittel wie z.B. Dichlormethan oder Toluol bei Temperaturen von -50 °C bis 150 °CV, gegebenenfalls unter Zusatz eines Katalysators wie z.B. Zinn(IV)-chlorid, Titan(IV)-chlorid, Zinn(IV)-triflat oder Zinkbromid, miteinander um.

Die Ester der Formel X können isoliert und in der voranstehend angegebenen Weise mit einem Cyanid zu den Nitrilen der Formel XI umgesetzt werden, sie können jedoch auch in situ, ohne daß man sie isoliert, gebildet und mit einem Cyanid zur Umsetzung gebracht werden.

Nitrile der Formel XI können mit Basen oder Säuren zu Säuren der Formel XII, in der $R^5$ H bedeutet, umgesetzt werden, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat oder Erdalkalimetallhydroxide wie Calciumhydroxid geeignet sind. Als Säuren sind beispielsweise Salzsäure, Schwefelsäure oder Bromwasserstoffsäure geeignet. Die Reaktionstemperatur liegt gewöhnlich zwischen -20 °C und +200 °C, vorzugsweise zwischen 0 °C und +100 °C. Bei diesen Temperaturen sind die Reaktionen in der Regel zwischen 30 Minuten und 48 Stunden beendet.

Mit Alkoholen $R^5$-OH können Nitrile der Formel XI unter Säurekatalyse zu Säurederivaten der Formel XII, in der $R^5$ niederes Alkyl, Cycloalkyl oder Aryl bedeutet, umgesetzt werden.

Geeignete Säuren sind insbesondere Chlorwasserstoff, Schwefelsäure und Bortrifluorid, beispielsweise in Form seines Diethyletheradduktes. Vorteilhafterweise führt man die Umsetzung in einem Überschuß des Alkohols $R^5$-OH als Lösungsmittel bei Temperaturen zwischen 0 °C und +150 °C durch.

Aus den Säurederivaten der Formel XII können die bekannten Lactone der Formel XIII erhalten werden, indem aus ersteren die Hydroxymethyl- (X = O) bzw. Mercaptomethyl (X = S) -gruppe wieder in Freiheit gesetzt wird und die so gewonnenen Zwischenprodukte der Formel XIIa,

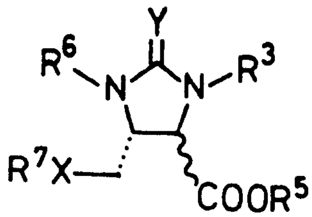

XIIa

worin X, Y, $R^3$ und $R^5$ die voranstehend angegebene Bedeutung besitzen, $R^6$ H oder $CHR^1R^2$ und $R^7$ H, Alkanyol oder Aroyl unter Wasser- ($R^5$ = H) bzw. Alkohol ($R^5 \neq$ H) -abspaltung cyclisiert werden.

Zur Spaltung der Bicyclen der Formel XII eignen sich beispielsweise Säuren wie Salzsäure oder Schwefelsäure, die, zweckmäßigerweise in einem Lösungsmittel, z.B. Wasser oder Alkoholen, zu den Hydantoinen der Formel XIIa, worin $R^6$ = $R^7$ = H, führen.

Als Spaltungsreagenz weiterhin anwendbar sind Schwermetallsalze wie Silbernitrat, Quecksilber(II)-chlorid oder Quecksilber(II)-acetat und die anschließende Zersetzung der intermediär gebildeten Metallmercaptide mit Schwefelwasserstoff. Für diese Spaltungsreaktion geeignete Lösungsmittel finden sich beispielsweise unter den Alkoholen wie Methanol, Ethanol, Amiden wie Dimethylformamid oder Ethern wie Tetrahydrofuran.

Mit Reduktionsmitteln können Säurederivate der Formel XII, in denen X S bedeutet, zu Zwischenprodukten der Formel XIIa, worin $R^6$ $CHR^1R^2$ und X S bedeutet, umgesetzt werden. Geeignete Reduktionsmittel sind beispielsweise Metalle wie Zink. Die Spaltung der Säurederivate durch Reduktion mit Metallen erfolgt vorzugsweise in saurem Milieu. Hierfür geeignete Säuren sind beispielsweise Mineralsäuren wie Salzsäure oder Schwefelsäure oder organische Säuren wie Ameisensäure oder Essigsäure. Zweckmäßig werden diese Säuren als Lösungsmittel oder im Gemisch mit anderen Lösungsmitteln wie z.B. Alkoholen verwendet. Die Reaktionstemperatur liegt üblicherweise zwischen 0 °C und 200 °C, vorzugsweise zwischen 20 °C und 150 °C. Bei diesen Temperaturen sind die Reaktionen in der Regel zwischen 15 Minuten und 24 Stunden beendet.

Unter den voranstehend angegebenen Bedingungen werden üblicherweise Verbindungen der Formel XIIa erhalten, worin $R^7$ H bedeutet. Wird die Reduktion jedoch in wasserfreiem Milieu unter Zusatz eines Acylierungsmittels, wie z.B. einem Säurehalogenid oder -anhydrid, durchgeführt, so gelangt man zu Verbindungen der Formel XIIa, in denen $R^7$ Alkanoyl oder Aroyl bedeutet. Derartige Verbindungen eignen sich bevorzugt zur Überführung in Lactone der Formel XIII.

Die erhaltenen Zwischenprodukte der Formel XIIa können isoliert werden; sie werden jedoch zweckmäßigerweise in situ weiter zu den bekannten Lactonen der Formel XIII umgesetzt. Häufig entstehen letztere Lactone bereits in hohem Maße oder vollständig bereits bei der Spaltung der Bicyclen der Formel XII. Zur Bildung der Lactone der Formel XIII aus den Zwischenprodukten der Formel XIIa werden diese mit einem die Wasser- oder Alkoholabspaltung bewirkenden Mittel behandelt. Hierfür geeignete Mittel stehen beispielsweise in Alkalimetallsalzen wie Kalium- oder Natriumacetat, Kaliumthioacetat, in Säuren wie Methansulfonsäure, p-Toluolsulfonsäure, Phosphorsäure, Schwefelsäure oder Salzsäure, in Anhydriden wie Acetanhydrid oder Trifluoracetanhydrid oder in anderen Dehydratisierungsreagenzien wie beispielsweise Aluminiumoxid, Kaliumhydroxid, Cyansäure oder Dicyclohexylcarbodiimid zur Verfügung. Die Anwendung eines Überschusses der vorbenannten Lactonisierungsmittel erweist sich oft als vorteilhaft, Geeignete Reaktionsbedingungen für die Lactonisierung lassen sich beispielsweise Houben-Weyl, Methoden der Organischen Chemie, Bd. E5, entnehmen.

Verbindungen der Formel XIIa, worin $R^7$ Alkanoyl oder Aroyl bedeutet, lassen sich vorteilhafterweise in Lactone der Formel XIII überführen, indem man deren entsprechende Alkalimetallsalze, insbesondere deren Natrium- oder Kaliumsalze (XIIa, $R^5$ = Na, K) einer thermischen Epimerisierung/Cyclisierung unterwirft. Man führt die Umsetzung der gegebenenfalls in situ hergestellten Alkalimetallsalze von Carbonsäuren der Formel XII vorteilhaft in einem inerten Lösungsmittel wie Dimethylformamid, Dimethylacetamid, Dimethylsulfoxid oder N-Methylpyrrolidon bei Temperaturen von 30-200 °C, insbesondere bei 50-150 °C, durch. Die Reaktionzeiten liegen bei 30 Minuten bis 12 Stunden.

Die Überführung der Lactone der Formel XIII in D-(+)-Biotin ist z.B. aus DE-20 58 234 und DE-23 31 244 bekannt.

In einem weiteren Verfahren zur Herstellung von D-(+)-Biotin aus (7aR)-1H,3H-Imidazo[1,5-c]azolen der Formel I werden Nitrile der Formel XI mit einer metallorganischen Verbindung zu Oxoverbindungen der Formel XIV umgesetzt. Hierfür geeignete metallorganische Verbindungen sind beispielsweise solche des Magnesiums (Verfahren nach Blaise), des Zinks (Verfahren nach Reformatzky), des Lithiums oder des

Aluminiums. Die Reaktionsbedingungen zur Umsetzung der metallorganischen Verbindungen orientieren sich an der Natur und chemischen Reaktivität der vorhandenen funktionellen Gruppen und können analog bekannten Verfahren gewählt werden, wie sie beispielsweise in Houben-Weyl, Methoden der Organischen Chemie, Bd. VII/2a, S. 603, beschrieben sind.

Zur Überführung der bicyclischen Oxoverbindungen der Formel XIV in die Hydantoine der Formel XV mittels einer Säure oder eines Reduktionsmittels eignen sich die gleichen, voranstehend angegebenen Methoden zur Darstellung der Hydantoine der Formel XIIa aus den Säurederivaten XII. Bei einer Spaltung mittels Säuren werden Imidazolidine der Formel XV erhalten, in denen $R^6$ H bedeutet, bei einer Spaltung durch ein Reduktionsmittel bedeutet $R^6$ in Formel XV $R^1R^2CH$. Die Reaktionsbedingungen orientieren sich an den zuvor angegebenen Methoden.

Verbindungen der Formel XV können unter basischen Bedingungen zu den Hemiacetalen der Formel XVI cyclisiert werden. Hierfür geeignete Basen sind beispielsweise Alkalimetallhydroxide wie Natrium- oder Kaliumhydroxid, Alkalimetallcarbonate wie Natriumhydrogencarbonat, Kaliumcarbonat, Kaliumhydrogencarbonat, Alkalimetallacetate wie Natrium- oder Kaliumacetat, Erdalkalimetallhydroxide und - oxide wie Calciumhydroxid, Calciumoxid oder Aluminiumoxid oder organische Basen wie Triethylamin, Pyridin, Lutidin, Piperidin, Morpholin, Piperazin, Collidin oder Chinolin. Weiterhin eignen sich auch basische Ionenaustauscher zur Cyclisierung von Verbindungen der Formel XV. Zweckmäßig führt man die Reaktion in einem inerten Lösungsmittel durch. Als inerte Lösungsmittel eignen sich vorzugsweise Kohlenwasserstoffe wie Cyclohexan, Benzol, Toluol, Ether wie Tetrahydrofuran, Dioxan, Amide wie Dimethylformamid, Hexamethylphosphorsäuretriamid, Sulfoxide wie Dimethylsulfoxid, Alkohole wie Methanol, Ethanol, Ester wie Ethylacetat oder niedere Carbonsäuren wie Ameisensäure oder Essigsäure. Auch ein Überschuß an organischer Base eignet sich als Lösungsmittel, welcher in Kombination mit einer niederen Carbonsäure ein besonders bevorzugtes Cyclisierungsmedium darstellt. Die Reaktionstemperaturen liegen zweckmäßig zwischen etwa 0° und 200 °C, vorzugsweise zwischen 20 und 150 °C, die Reaktionszeiten zwischen etwa 1 und 48 Stunden.

Hemiacetale der Formel XVI sind bekannt und können nach bekannten Methoden, wie beispielsweise der DE-20 58 234 zu entnehmen, zu D-(+)-Biotin umgesetzt werden.

In einem weiteren Verfahren zur Herstellung von D-(+)-Biotin aus (7aR)-1H,3H-Imidazo[1,5-c]azolen der Formel I werden Nitrile der Formel XI mit einem Reduktionsmittel zu einem Aldehyd der Formel XVII umgesetzt. Hierfür geeignete Verfahren sind beispielsweise Houben-Weyl, Methoden der Organischen Chemie, Bde. 7/1, S. 299 und E3, S. 476, zu entnehmen. Als Reduktionsmittel können z.B. Zinn(II)-chlorid mit Chlorwasserstoff in etherischer Lösung oder Wasserstoff unter Raney-Nickel Katalyse in saurem Milieu Anwendung finden. Gute Ergebnisse sind auch durch Umsetzung der Nitrile der Formel XI mit 2-Mercaptoethanol zu den entsprechenden 2-substituierten 1,3-Benzooxathiazolen und deren Reduktion mittels Natriumborhydrid zu erzielen. Besonders vorteilhaft erfolgt die Reduktion der Nitrile der Formel XI mit komplexen Hydriden wie Natrium-hydro-triethoxyaluminat, Lithium-hydro-triethoxyalanat oder, insbesondere bevorzugt, mit Diisobutylaluminiumhydrid.

Man führt die Umsetzung der komplexen Hydride mit den Nitrilen der Formel XI vorteilhaft in einem inerten Lösungsmittel durch. Als inerte Lösungsmittel eignen sich vorzugsweise Kohlenwasserstoffe wie Pentan, Hexan, Cyclohexan, Benzol oder Toluol oder Ether wie Diethylether, Tetrahydrofuran, Dioxan oder Ethylenglykoldimethylether sowie Gemische dieser Lösungsmittel untereinander. Die Reaktionstemperaturen liegen zweckmäßig zwischen etwa -120 °C und +150 °C, vorzugsweise zwischen -80 °C und +100 °C, die Reaktionszeiten zwischen etwa 30 Minuten und 24 Stunden.

Aus den Aldehyden der Formel XVII lassen sich mit geeigneten Phosphororganylen in Anwesenheit einer Base die ungesättigten Verbindungen der Formel XVIII herstellen. So können beispielsweise Phosphorane nach Überführung in die entsprechenden Ylide nach den in Houben-Weyl, Methoden der Organischen Chemie, Bd. 5/16, S. 383, Phosphonate nach Bildung des Anions in analog den z.B. in Organic Reactions, Bd. 25, John Wiley, New York, 1978, Kap. 2, beschriebenen Verfahren oder Phosphinoxide auf beispielsweise in Houben-Weyl, Methoden der organischen Chemie, Bd. XII/1, S. 167, beschriebene Weise für die Olefinierung von Aldehyden der Formel XVII Verwendung finden. Als Basen zur Bereitung eines reaktiven Phosphorylids, Phosphonat- oder Phosphinoxidanions eignen sich in Abhängigkeit von der Deprotonierungsfähigkeit des eingesetzten Phosphororganyls z.B. Alkalimetallhydroxide wie Natrium- oder Kaliumhydroxid, Alkalimetallcarbonate wie Natrium- oder Kaliumcarbonat, Alkoholate wie Natriummethylat, Natriumethylat, Lithiumethylat oder Kalium-tert.-butanolat, Alkalimetallamide wie Kalium- oder Natriumamid, Alkalimetallorganyle wie Methyllithium, Butyllithium oder Phenyllithium oder andere organische Basen wie Lithiumdiisopropylamid oder Natriummethylsulfinylmethylid. Zweckmäßig führt man die Reaktion in einem inerten Lösungsmittel durch. Als inerte Lösungsmittel eignen sich vorzugsweise Ether wie Diethylether, Ethylenglykoldimethylether, Tetrahydrofuran oder Dioxan sowie Amide wie Dimethylformamid, Hexamethylphosphorsäuretriamid, Dimethylacetamid oder N-Methylpyrrolidon, ferner Sulfoxide wie Dimethylsulfoxid oder Sulfolan sowie Kohlenwasserstoffe wie Pentan, Hexan, Cyclohexan, Benzol oder Toluol. Die Reaktionstemperaturen liegen zweckmäßig entsprechend der Reaktivität des eingesetzten Phosphororganyls zwischen etwa -10 °C und etwa +150 °C, vorzugsweise zwischen +20 °C und +100 °C, die Reaktionszeiten zwischen 1 und 48 Stunden.

Nach zur Spaltung von Säurederivaten der Formel XII analogen, voranstehend beschriebenen verfahren werden die ungesättigten bicyclischen Verbindungen der Formel XVIII mit Säuren und/oder Reduktionsmitteln umgesetzt. Als Zwischenprodukte entstehende Hydroxy-(X = O) oder Mercapto- (X = S)-methylhydantoine erfahren unter den Spaltungsbedingungen Ringschluß zu den Biotinderivaten der Formel XIX, worin im Falle einer Säurespaltung $R^6$ H bedeutet; bei einer Spaltung durch ein Reduktionsmittel bedeutet $R^6$ $R^1R^2CH$. Die Reaktionsbedingungen orientieren sich an den zuvor angegebenen Methoden.

Schließlich können Oxoverbindungen der Formel XIV nach zur Spaltung von Säurederivaten der Formel XII analogen, voranstehend beschriebenen Verfahren in Biotinderivate der Formel XX überführt werden, wobei $R^3$, X, Y und Z die angegebene Bedeutung zukommt.

Biotinderivate der Formel XIX sind bekannt und können nach bekannten Methoden wie z.B. in DE-20 58 234, EP-O 036 030 und EP-O 084 377 offenbart, zu D-(+)-Biotin umgesetzt werden.

Das erfindungsgemäße Verfahren erlaubt somit die Herstellung von optisch aktivem D-(+)-Biotin auf einfache und stereospezifische Weise in hohen Ausbeuten aus leicht zugänglichen, wohlfeilen Ausgangsstoffen in wenigen, zum Teil in Eintopfverfahren durchführbaren Syntheseschritten und stellt damit einen wesentlichen Fortschritt auf dem Gebiet der Biotinsynthese dar.

Beispiel 1

Eine unter Stickstoff gehaltene Lösung von 97,32 g (0,3 Mol) (7aR)-3-Phenyl-6-benzyl-1H,3H-imidazo-[1,5-c]thiazol-5,7(6H,7aH)-dion in 800 ml Tetrahydrofuran wird bei +5 °C portionsweise mit 6,53 g (0,3 Mol) Lithiumborhydrid versetzt und anschließend 3 Stunden bei Raumtemperatur gerührt.

Danach zersetzt man vorsichtig mit 400 ml Eiswasser, destilliert das Tetrahydrofuran weitgehend ab und extrahiert das Produkt mit insgesamt 800 ml Ethylacetat.

Die organischen Phasen werden vereinigt, zweimal mit je 150 ml Wasser gewaschen, über Natriumsulfat getrocknet und unter vermindertem Druck eingeengt.

Man erhält 90,8 g (7RS,7aR)-3-Phenyl-6-benzyl-7,7a-dihydro-7-hydroxy-1H,3H-imidazo[1,5-c]thiazol-5-(6H)-on mit einem Schmelzpunkt von 151-153 °C.

$$[\alpha]_{365}^{20} = -875°,$$

c = 1 (Methanol)

Beispiel 2

In eine Lösung von 324,4 g (1 Mol) (7aR)-3-Phenyl-6-benzyl-1H,3H-imidazo[1,5-c]thiazol-5,7(6H,7aH)-dion in 1200 ml Tetrahydrofuran läßt man bei Raumtemperatur eine Lösung von 43,35 g (1,1 Mol) 96 %igem Natriumborhydrid in 300 ml Wasser einfließen und rührt das Reaktionsgemisch 12 Stunden bei 50 °C. Anschließend kühlt man auf 20 °C ab, trennt die wässrige Phase ab und engt die organische Lösung unter vermindertem Druck ein.

Den Rückstand suspendiert man in 1000 ml 2-Propanol, destilliert 100 ml Lösungsmittel ab und läßt bei 0 °C kristallisieren.

Man erhält 303,5 g (7R,7aR)-3-Phenyl-6-benzyl-7,7a-dihydro-7-hydroxy-1H,3H-imidazo[1,5-c]thiazol-5-(6H)-on mit einem Schmelzpunkt von 174-175 °C.

$$[\alpha]_{365}^{20} = -1026°,$$

c = 1 (Methanol)

Beispiel 3

In eine Lösung von 324,4 g (1 Mol) (7aR)-3-Phenyl-6-benzyl-1H,3H-imidazo[1,5-c]thiazol-5,7(6H,7aH)-dion in 1200 ml Tetrahydrofuran läßt man bei Raumtemperatur eine Lösung von 43,35 g (1,1 Mol) 96 %igem Natriumborhydrid in 300 ml Wasser einfließen und rührt das Reaktionsgemisch 7 Stunden bei 50 °C. Anschließend kühlt man auf 20 °C ab, trennt die wäßrige Phase ab und engt die organische Lösung unter vermindertem Druck ein.

Den Rückstand suspendiert man in 1000 ml 2-Propanol, destilliert 100 ml Lösungsmittel ab und läßt bei 0 °C kristallisieren.

Man erhält 298,7 g (7RS,7aR)3-Phenyl-6-benzyl-7,7a-dihydro-7-hydroxy-1H,3H-imidazo[1,5-c]thiazol-5-(6H)-on mit einem Schmelzpunkt von 164-166 °C.

$$[\alpha]_{365}^{20} = -964°,$$

c = 1 (Methanol)

Beispiel 4

In eine unter Stickstoff gehaltene Suspension von 97,92 g (0,3 Mol) (7RS,7aR)-3-Phenyl-6-benzyl-7,7a-dihydro-7-hydroxy-1H,3H-imidazo[1,5-c]thiazol-5(6H)-on in 300 ml Acetonitril werden innert 30 Minuten 50,27 g (0,31 Mol) 1,1-Carbonyldiimidazol bei +10 °C eingetragen. Man rührt die entstehende klare Lösung 30 Minuten bei Raumtemperatur, engt ein und verteilt den Rückstand zwischen 300 ml Wasser und 500 ml Ethylacetat. Die organische Phase wird dreimal mit je 100 ml Wasser gewaschen, über Natriumsulfat getrocknet und unter vermindertem Druck eingeengt.

Man erhält 104,6 g (7RS,7aR)-3-Phenyl-6-benzyl-7,7a-dihydro-7-(imidazol-1-ylcarbonyloxy)-1H,3H-imidazo-[1,5-c]thiazol-5(6H)-on als Öl.

$$[\alpha]_{365}^{20} = -750°,$$

c = 1 (Methanol)

Beispiel 5

In eine Suspension von 0,96 g (32 mMol) 80 %igem Natriumhydrid in 20 ml Tetrahydrofuran tropft man unter Stickstoff bei Raumtemperatur eine Lösung von 9,79 g (30 mMol) (7RS,7aR)-3-Phenyl-6-benzyl-7,7a-dihydro-7-hydroxy-1H,3H-imidazo[1,5-c]thiazol-5(6H)-on in 100 ml Tetrahydrofuran und rührt die entstehende Lösung 45 Minuten bei Raumtemperatur. Anschließend wird eine Lösung von 6,34 g (32 mMol) 1,1-Sulfonyldiimidazol in 80 ml Tetrahydrofuran bei Raumtemperatur innert 10 Minuten zugetropft und das Gemisch weitere 20 Minuten gerührt.

Man engt ein, versetzt den Rückstand mit 200 ml Eiswasser und extrahiert dreimal mit je 100 ml Diethylether. Die vereinigten organischen Phasen werden über Natriumsulfat getrocknet und unter vermindertem Druck eingeengt.

Nach Chromatographie des Rückstandes an Kieselgel (Ethylacetat) erhält man 8,1 g (7RS,7aR)-3-Phenyl-6-benzyl-7,7a-dihydro-7-(imidazol-1-ylsulfonyloxy)-1H,3H-imidazo[1,5-c]thiazol-5(6H)-on als Öl.

$$[\alpha]_{365}^{20} = -710°,$$

c = 1 (Methanol)

Beipiel 6

In eine Lösung von 97,92 g (0,3 Mol) (7RS,7aR)-3-Phenyl-6-benzyl-7,7a-dihydro-7-hydroxy-1H,3H-imidazo-[1,5-c]thiazol-5(6H)-on in 900 ml Pyridin tropft man innert 10 Minuten 33,69 g (0,33 Mol) Essigsäureanhydrid und rührt die Lösung 12 Stunden bei Raumtemperatur.

Anschließend engt man unter vermindertem Druck ein, löst den Rückstand in 600 ml Toluol, wäscht dreimal mit je 100 ml 1 N Salzsäure und destilliert das Lösungsmittel ab.

Man erhält 110,1 g (7RS,7aR)-3-Phenyl-6-benzyl-7,7a-dihydro-7-acetoxy-1H,3H-imidazo[1,5-c]thiazol-5-(6H)-on als Öl.

$$[\alpha]_{365}^{20} = -786°,$$

c = 1 (Methanol)

Beispiel 7

In eine Lösung von 210,24 g (0,5 Mol) (7RS,7aR)-3-Phenyl-6-benzyl-7,7a-dihydro-7-(imidazol-1-ylcarbonyloxy)-1H,3H-imidazo[1,5-c]thiazol-5(6H)-on in 1500 ml Aceton tropft man innert 10 Minuten 354,85 g (2,5 Mol) Iodmethan und rührt die Lösung 3 Stunden bei Raumtemperatur.

Anschließend wird eingeengt, der Rückstand in 750 ml Dimethylformamid gelöst, mit 98,02 g (2 Mol) Natriumcyanid versetzt und das Gemisch eine Stunde bei 75 °C gerührt.

Danach wird die Lösung in 5000 ml Eiswasser eingerührt und fünfmal mit je 500 ml Toluol extrahiert. Die vereinigten organischen Phasen werden mit Wasser sowie gesättigter Kochsalz-Lösung gewaschen, über Natriumsulfat getrocknet und unter vermindertem Druck eingeengt.

Man erhält 150,7 g eines Gemisches aus (7R,7aR)- und (7S,7aR)-3-Phenyl-6-benzyl-7-cyano-7,7a-dihydro-1H,3H-imidazo[1,5-c]thiazol-5(6H)-on als öl, das an Kieselgel mit Petrolether/tert.-Butylmethylether (1:1, v/v) chromatographiert wird.

7S-Nitril: Ausbeute 35 g; Fp.: 102-103 °C

$[\alpha]_{365}^{20} = -795°$, c = 1 (Methanol)

7R-Nitril: Ausbeute 107 g; Fp.: 109-110 °C

$[\alpha]_{365}^{20} = -840°$, c = 1 (Methanol)

Beispiel 8

45,65 g (0,1 Mol) (7RS,7aR)-3-Phenyl-6-benzyl-7,7a-dihydro-7-(imidazol-1-sulfonyloxy)-1H,3H-imidazo-[1,5-c]thiazol-5(6H)-on werden in 350 ml Aceton vorgelegt. In diese Lösung tropft man innert 5 Minuten 60,49 g (0,5 Mol) Allylbromid und rührt das Reaktionsgemisch 30 Minuten bei Raumtemperatur.

Anschließend wird eingeengt, der Rückstand in 100 ml 1-Methyl-2-pyrrolidon gelöst, die Lösung mit 19,6 g (0,4 Mol) Natriumcycanid versetzt und 45 Minuten bei 80 °C gerührt.

Danach wird das Reaktionsgemisch in 1000 ml Eiswasser eingerührt und dreimal mit je 200 ml Toluol extrahiert. Die vereingten organischen Phasen wäscht man mit Wasser und gesättigter Kochsalzlösung, trocknet über Natriumsulfat und engt unter vermindertem Druck ein.

Man erhält 16,8 g eines Gemisches aus (7R,7aR)- und (7S,7aR)-3-Phenyl-6-benzyl-7-cyano-7,7a-dihydro-1H,3H-imidazo[1,5-c]thiazol-5(6H)-on als Öl, das an Kieselgel mit Petrolether/tert.-Butylmethylether (1:1, v/v chromatographiert wird.

7S-Nitril: Ausbeute 3,8 g; Fp.: 103 °C

$[\alpha]_{365}^{20} = -790°$

7R-Nitril: Ausbeute 11,8 g; Fp.: 109-110 °C

$[\alpha]_{365}^{20} = -845°$

c = 1 (Methanol)

Beispiel 9

In eine unter Stickstoff gehaltene Lösung von 368,45 g (1 Mol) (7RS,7aR)-3-Phenyl-6-benzyl-7,7a-dihydro-7-acetoxy-1H,3H-imidazo[1,5-c]thiazol-5(6H)-on in 3500 ml Dichlormethan läßt man bei -20 °C 104,17 g (1,05 Mol) Trimethylsilylcyanid einfließen, tropft anschließend unter kräftigem Rühren 189,71 g (1 Mol) Titan(IV)-chlorid innert 15 Minuten zu und rührt 45 Minuten bei -20 °C.

Das rote Reaktionsgemisch wird bei 0 °C vorsichtig mit 1000 ml 1 N Salzsäure versetzt und bis zur vollständigen Entfärbung bei Raumtemperatur gerührt.

Anschließend wird die organische Phase abgetrennt, zweimal mit je 1000 ml Wasser gewaschen, über Natriumsulfat getrocknet und eingeengt.

Man erhält 321,3 g (7RS,7aR)-3-Phenyl-6-benzyl-7-cyano-7,7a-dihydro-1H,3H-imidazo[1,5-c]thiazol-5-(6H)-on als Öl.

$$[\alpha]_{365}^{20} = -823°,$$

c = 1 (Methanol)

Beispiel 10

Eine Suspension von 97,92 g (0,3 Mol) (7RS,7aR)-3-Phenyl-6-benzyl-7,7a-dihydro-7-hydroxy-1H,3H-imidazo[1,5-c]thiazol-5(6H)-on in 1200 ml Toluol wird mit 31,64 g (0,4 Mol) Pyridin und 33,69 g (0,33 Mol) Essigsäureanhydrid versetzt und 10 Stunden bei 50 °C gerührt.

Man kühlt die Lösung auf Raumtemperatur ab, wäscht dreimal mit je 200 ml 1 N Salzsäure und destilliert 200 ml Toluol ab.

Nach Abkühlung auf -20 °C versetzt man unter Stickstoff mit 30,76 g (0,31 Mol) Trimethylsilylcyanid und tropft innert 5 Minuten 56,91 g (0,3 Mol) Titan(IV)-chlorid zu.

In das braune Reaktionsgemisch tropft man bei 0 °C vorsichtig 350 ml 1 N Salzsäure und rührt bis zur vollständigen Entfärbung bei Raumtemperatur.

Die organische Phase wird abgetrennt, zweimal mit je 300 ml Wasser gewaschen und eingeengt.

Man erhält 89,6 g (7RS,7aR)-3-Phenyl-6-benzyl-7-cyano-7,7a-dihydro-1H,3H-imidazo[1,5-c]thiazol-5(6H)-on als Öl.

$$[\alpha]_{365}^{20} = -817°,$$

c = 1 (Methanol)

Beispiel 11

Eine Lösung von 97,92 g (0,3 Mol) (7RS,7aR)-3-Phenyl-6-benzyl-7,7a-dihydro-7-hydroxy-1H,3H-imidazo[1,5-c]thiazol-5(6H)-on in 960 ml Dichlormethan wird mit 47,46 g (0,6 Mol) Pyridin und 33,69 g (0,33

Mol) Essigsäureanhydrid versetzt und 8 Stunden unter Rückfluß gekocht.

Man kühlt auf Raumtemperatur ab, wäscht dreimal mit je 300 ml 1 N Salzsäure und trocknet über Natriumsulfat.

Anschließend kühlt man die Lösung auf -20 °C ab, läßt unter Stickstoff 30,76 g (0,31 Mol) Trimethylsilylcyanid zufließen und tropft innert 5 Minuten 78,15 g (0,3 Mol) Zinn(IV)-chlorid zu.

Danach versetzt man das Reaktionsgemisch bei 0 °C vorsichtig mit 350 ml 1 N Salzsäure und rührt bei Raumtemperatur bis zur vollständigen Entfärbung.

Die organische Phase wird abgetrennt, zweimal mit je 300 ml Wasser gewaschen, über Natriumsulfat getrocknet und eingeengt.

Man erhält 86,3 g (7RS,7aR)-3-Phenyl-6-benzyl-7-cyano-7,7a-dihydro-1H,3H-imidazo[1,5-c]thiazol-5(6H)-on als Öl.

$$[\alpha]^{20}_{365} = -826°,$$

c = 1 (Methanol)

Analog werden hergestellt

(7RS,7aR)-3-Phenyl-6-(4-methoxybenzyl)-7-cyano-7,7a-dihydro-1H,3H-imidazo[1,5c]thiazo-5(6H)-on
(7RS,7aR)-3-Phenyl-6-(4-chlorbenzyl)-7-cyano-7,7a-dihydro-1H,3H-imidazo[1,5c]thiazo-5(6H)-on
(7RS,7aR)-3-Phenyl-6-(4-nitrobenzyl)-7-cyano-7,7a-dihydro-1H,3H-imidazo[1,5c]thiazo-5(6H)-on
(7RS,7aR)-3-Phenyl-6-(trimethylsilyl)-7-cyano-7,7a-dihydro-1H,3H-imidazo[1,5c]thiazo-5(6H)-on
(7RS,7aR)-3-Phenyl-6-(2-propenyl)-7-cyano-7,7a-dihydro-1H,3H-imidazo[1,5c]thiazo-5(6H)-on
(7RS,7aR)-3-Propyl-6-benzyl-7-cyano-7,7a-dihydro-1H,3H-imidazo[1,5c]thiazo-5(6H)-on
(7RS,7aR)-3,3-Dimethyl-6-benzyl-7-cyano-7,7a-dihydro-1H,3H-imidazo[1,5c]thiazo-5(6H)-on
(7RS,7aR)-3-Ethyl-3-methyl-6-benzyl-7-cyano-7,7a-dihydro-1H,3H-imidazo[1,5c]thiazo-5(6H)-on
(7RS,7aR)-3-Cyclohexyl-6-benzyl-7-cyano-7,7a-dihydro-1H,3H-imidazo[1,5c]thiazo-5(6H)-on
(7RS,7aR)-3,3-Tetramethylenyl-6-benzyl-7-cyano-7,7a-dihydro-1H,3H-imidazo[1,5c]thiazo-5(6H)-on
(7RS,7aR)-3,3-Pentamethylenyl-6-benzyl-7-cyano-7,7a-dihydro-1H,3H-imidazo[1,5c]thiazo-5(6H)-on

Beispiel 12

Eine Lösung von 13,42 g (40 mMol) eines Gemisches aus (7R,7aR)- und (7S,7aR)-3-Phenyl-6-benzyl-7-cyano-7,7a-dihydro-1H,3H-imidazo[1,5-c]thiazol-5(6H)-on in 75 ml n-Butanol wird mit einer Lösung von 7,92 g (0,12 Mol) 85 %igem Kaliumhydroxid in 20 ml Wasser versetzt und 90 Minuten bei 100 °C gerührt.

Anschließend destilliert man das Lösungsmittel weitgehend ab, versetzt mit 200 ml Wasser und extrahiert dreimal mit je 100 ml Dichlormethan.

In die wässrige Phase tropft man bei 0 °C konz. Salzsäure, bis pH 1 erreicht ist, saugt das Produkt ab und wäscht portionsweise mit insgesamt 100 ml Wasser nach.

Man erhält 13,5 g (7R,7aR)-3-Phenyl-5(6H)-oxo-6-benzyl-7,7a-dihydro-1H,3H-imidazo[1,5]thiazol-7-carbonsäure mit einem Schmelzpunkt von 184-185 °C.

$$[\alpha]^{20}_{365} = -960°,$$

c = 1 (Methanol)

Beispiel 13

Man suspendiert 134,17 g (0,4 Mol) (7R,7aR)-3-Phenyl-6-benzyl-7-cyano-7,7a-dihydro-1H,3H-imidazo-[1,5-c]thiazol-5(6H)-on in 1000 ml konz. Salzsäure und rührt das Gemisch 3 Stunden bei 80 °C. Anschließend wird auf 0 °C abgekühlt, 3 Stunden nachgerührt, abgesaugt und portionsweise mit 500 ml Wasser gewaschen.

Man erhält 129,8 g (7R,7aR)-3-Phenyl-5(6H)-oxo-6-benzyl-7,7a-dihydro-1H,3H-imidazo[1,5-c]thiazol-7-carbonsäure mit einem Schmelzpunkt von 183-184 °C.

$$[\alpha]_{365}^{20} = -950°,$$

c = 1 (Methanol)

Beispiel 14

Nach der in Beispiel 13 angegebenen Arbeitsweise werden 13,42 g (40 mMol) (7S,7aR)-3-Phenyl-6-benzyl-7-cyano-7,7a-dihydro-1H,3H-imidazo[1,5-c]thiazol-5(6H)-on und 100 ml konz. Salzsäure 3 Stunden bei 80 ° zur Reaktion gebracht. Nach der angegebenen Aufarbeitung erhält man (7S,7aR)-3-Phenyl-5(6H)-oxo-6-benzyl-7,7a-dihydro-1H, 3H-imidazo[1,5-c]thiazol-7-carbonsäure; Fp.: 220-225 °C.

Beispiel 15

Nach der in Beispiel 13 angegebenen Arbeitsweise werden 134,17 g (0,4 Mol) eines Gemisches aus (7R,7aR)- und (7S,7aR)-3-Phenyl-6-benzyl-7-cyano-7,7a-dihydro-1H,3H-imidazo[1,5-c]thiazol-5(6H)-on mit Salzsäure hydrolysiert. Man erhält 127,2 g eines Gemisches aus (7R,7aR)- und (7S,7aR)-3-Phenyl-5(6H)-oxo-6-benzyl-7,7a-dihydro-1H,3H-imidazo[1,5-c]thiazol-7-carbonsäure, welches keinen definierten Schmelzpunkt aufweist.

Beispiel 16

Zu einer Lösung von 20,04 g (0,11 Mol) 1,1-Sulfinyldiimidazol (W. Walter, M. Radke; Liebigs Ann. Chem. 1979, 1756-67) in 350 ml Dichlormethan werden unter Stickstoff bei Raumtemperatur 32,64 g (0,1 Mol) (7$\overline{RS,7}$aR)-3-Phenyl-6-benzyl-7-cyano-7,7a-dihydro-7-hydroxy-1H,3H-imidazo[1,5-c]thiazol-5(6H)-on gegeben.

Man rührt die klare Lösung 30 Minuten bei Raumtemperatur, wäscht zweimal mit je 150 ml Wasser, trocknet über Natriumsulfat und filtriert.

Zum Filtrat tropft man bei 10 °C 37,84 g (0,3 Mol) Dimethylsulfat (10 Minuten) und rührt die Lösung 4 Stunden bei 20 °C.

Anschließend zerstört man überschüssiges Dimethylsulfat durch Zugabe von Triethylamin bei 20 °C und engt unter vermindertem Druck ein.

Den öligen Rückstand löst man in 100 ml Dimethylformamid, trägt 14,7 g (0,3 Mol) Natriumcyanid ein und rührt das Reaktionsgemisch 60 Minuten bei 85 °C.

Danach wird das Reaktionsgemisch in 500 ml Eiswasser eingerührt und dreimal mit je 150 ml Ethylacetat extrahiert. Die vereinigten organischen Phasen wäscht man mit Wasser und gesättigter Kochsalzlösung und engt unter vermindertem Druck ein. Die Hydrolyse des erhaltenen Nitrils erfolgt analog der in Beispiel 6 beschriebenen Weise unter Verwendung von 200 ml n-Butanol, 19,8 g (0,3 Mol) 85 %igem Kaliumhydroxid und 40 ml Wasser.

Man erhält 22,68 g (7R,7aR)-3-Phenyl-5(6H)-oxo-6-benzyl-7,7a-dihydro-1H,3H-imidazo[1,5-c]thiazol-7-carbonsäure, Fp.: 180-181 °C,

$$[\alpha]_{365}^{20} = -945°,$$

c = 1, Methanol.

Beispiel 17

Eine Lösung von 9,92 g (28 mMol) (7R,7aR)-3-Phenyl-5(6H)-oxo-6-benzyl-7,7a-dihydro-1H,3H-imidazo-[1,5-c]thiazol-7-carbonsäure in 150 ml wasserfreier Essigsäure wird bei 80 °C portionsweise mit 7,32 g (112 mMol) Zinkpulver versetzt, weitere 4 Stunden bei 80 °C gerührt, filtriert und unter vermindertem Druck eingeengt.

Den Rückstand löst man in 500 ml Ethylacetat, wäscht mit 200 ml Wasser und 100 ml gesättigter Kochsalzlösung, trocknet über Natriumsulfat und engt unter vermindertem Druck ein. Beim Verreiben mit Diethylether kristallisiert das Produkt.

Man erhält 9,39 g (4R,5R)-1,3-Dibenzyl-2-oxo-5-mercaptomethyl-imidazolidin-4-carbonsäure mit einem Schmelzpunkt von 167-169 °C.

$$[\alpha]^{20}_{365} = +60°,$$

c = 1 (Methanol)

Beispiel 18

Eine Lösung von 9,92 g (28 mMol) (7R,7aR)-3-Phenyl-5-(6H) -oxo-6-benzyl-7,7a-dihydro-1H,3H-imidazo-[1,5-c]-thiazol-7-carbonsäure in einem Gemisch aus 100 ml wasserfreier Essigsäure und 4,29 g (42 mMol) Essigsäureanhydrid wird bei 90 °C portionsweise mit 7,32 g (112 mMol) Zinkpulver versetzt und danach 2 Stunden bei 110 °C gerührt.

Anschließend wird auf 60 °C abgekühlt, filtriert und unter vermindertem Druck eingeengt.

Den Rückstand suspendiert man in 120 ml Wasser, rührt 60 Minuten bei Raumtemperatur, filtriert und trocknet im Vakuum.

Man erhält 9,5 g (4R,5R)-1,3-Dibenzyl-2-oxo-5-acetylmercaptomethyl-imidazolin-4-carbonsäure mit einem Schmelzpunkt von 135-140 °C.

$$[\alpha]^{20}_{365} = +173°,$$

c = 1 (Methanol)

Beispiel 19

Man löst 35,64 g (0,1 Mol) (4R,5R)-1,3-Dibenzyl-2-oxo-5-mercaptomethyl-imidazolin-4-carbonsäure in 300 ml Dimethylacetamid, trägt 13,61 g (0,1 Mol) Natriumacetat-Trihydrat ein und erhitzt das Gemisch 40 Minuten auf 150 °C.

Anschließend wird auf Raumtemperatur abgekühlt, in 900 ml Wasser eingerührt und viermal mit je 100 ml Toluol extrahiert. Die vereinigten organischen Phasen werden dreimal mit je 50 ml Wasser und einmal mit 50 ml gesättigter Kochsalzlösung gewaschen und unter vermindertem Druck eingeengt. Der Rückstand wird aus Ethylacetat umkristallisiert.

Man erhält 28,76 g (3aS,6aR)-1,3-Dibenzyl-tetrahydrothieno[3,4-d]imidazol-2(3H),4-dion mit einem Schmelzpunkt von 118-119 °C.

$[\alpha]^{20}_{D}$ = +90,8°, c = 1 ($CHCl_3$)

Beispiel 20

Man löst 39,85 g (0,1 Mol) (4R,5R)-1,3-Dibenzyl-2-oxo-5-acetylmercaptomethyl-imidazolin-4-carbonsäure in 100 ml Dimethylformamid, trägt 11,42 g (0,1 Mol) Kaliumthioacetat ein und erhitzt das Reaktionsgemisch 2 Stunden auf 130 °C.

Anschließend wird auf Raumtemperatur abgekühlt, in 800 ml Wasser eingerührt und viermal mit je 100 ml Toluol extrahiert. Die vereinigten organischen Phasen werden dreimal mit je 50 ml Wasser und einmal mit gesättigter Kochsalzlösung gewaschen und unter vermindertem Druck eingeengt.

Den Rückstand kristallisiert man aus 2-Propanol um und erhält 25,4 g (3aS,6aR)-1,3-Dibenzyl-tetrahydrothieno[3,4-d]imidazol-2(3H),4-dion mit einem Schmelzpunkt von 118 °C.

$[\alpha]^{20}_{D}$ = +90,7 °, C = 1 ($CHCl_3$)

Beispiel 21

Eine Lösung von 39,85 g (0,1 Mol) (4R,5R)-1,3-Dibenzyl-2-oxo-5-acetylmercaptomethyl-imidazolin-4-carbonsäure in 300 ml 1N Natronlauge wird 60 Minuten bei Raumtemperatur gerührt, anschließend mit konz. Salzsäure auf pH 4 angesäuert und dreimal mit je 100 ml Dichlormethan extrahiert. Die vereinigten organischen Phasen werden über Natriumsulfat getrocknet und unter vermindertem Druck eingeengt.

Den Rückstand löst man in 100 ml N-Methylpyrrolidon, trägt 11,42 g (0,1 mol) Kaliumthioacetat ein und erhitzt das Reaktionsgemisch 90 Minuten auf 130 °C.

Anschließend wird auf Raumtemperatur abgekühlt, in 800 ml Wasser eingerührt und viermal mit je 100 ml Toluol extrahiert. Die vereinigten organischen Phasen werden dreimal mit je 50 ml Wasser und einmal mit gesättigter Kochsalzlösung gewaschen und unter vermindertem Druck eingeengt.

Den Rückstand kristallisiert man aus 2-Propanol um und erhält 22,6 g (3aS,6aR)-1,3-Dibenzyl-tetrahydrothieno-[3,4-d]imidazol-2(3H),4-dion mit einem Schmelzpunkt von 119-120 °C.

$[\alpha]_D^{20}$ = + 91,4°, c = 1 ($CHCl_3$)

Beispiel 22

Eine Lösung von 39,85 g (0,1 Mol) (4S,5R)-1,3-Dibenzyl-2-oxo-5-acetylmercaptomethyl-imidazolin-4-carbonsäure in 200 ml Methanol wird mit 100 ml einmolarer methanolischer Kalilauge versetzt und unter vermindertem Druck eingeengt.

Den öligen Rückstand löst man in 100 ml Dimethylformamid und erhitzt 3 Stunden auf 130 °C.

Anschließend wird auf Raumtemperatur abgekühlt, in 800 ml Wasser eingerührt und viermal mit je 100 ml Toluol extrahiert. Die vereinigten organischen Phasen werden dreimal mit je 50 ml Wasser und einmal mit gesättigter Kochsalzlösung gewaschen und unter vermindertem Druck eingeengt.

Den Rückstand kristalliert man aus 2-Propanol um und erhält 27,7 g (3aS,6aR)-1,3-Dibenzyltetrahydrothieno[3,4-d] imidazol-2(3H)-4-dion mit einem Schmelzpunkt von 119 °C.

$[\alpha]_D^{20}$ = +91,1°, c = 1 ($CHCl_3$)

Beispiel 23

Man löst 39,85 g (0,1 Mol) (4R,5R)-1,3-Dibenzyl-2-oxo-5-acetylmercaptomethyl-imidazolin-4-carbonsäure in 100 ml N-Methylpyrrolidon, trägt 11,22 g (0,2 Mol) Kalium-tert.-butylat ein und erhitzt das Reaktionsgemisch 2 Stunden auf 130 °C.

Anschließend wird auf Raumtemperatur abgekühlt, in 800 ml Wasser eingerührt und viermal mit je 100 ml Toluol extrahiert` Die vereinigten organischen Phasen werden dreimal mit je 50 ml Wasser gewaschen und unter vermindertem Druck eingeengt.

Den Rückstand kristallisiert man aus 2-Propanol um und erhält 28,3 g (3aS,6aR)-1,3-Dibenzyltetrahydrothieno[3,4-d]imidazol-2(3H),4-dion mit einem Schmelzpunkt von 118-119 °C.

$[\alpha]_D^{20}$ = +90,8°, c = 1 ($CHCl_3$)

Beispiel 24

Nach der in Beispiel 23 angegebenen Arbeitsweise werden 39,85 g (0,1 Mol) (4R,5R)-1,3-Dibenzyl-2-oxo-5-acetylmercaptomethyl-imidazolin-4-carbonsäure mit 5,40 g (0,1 Mol) Natriummethylat in 100 ml N-Methylpyrrolidon umgesetzt.

Man erhält 28,0 g (3aS,6aR)-1,3-Dibenzyltetrahydrothieno[3,4-d]imidazol-2(3H),4-dion mit einem Schmelzpunkt von 119-120 °C.

$[\alpha]_D^{20}$ = +91,3°, c = 1 ($CHCl_3$)

Beispiel 25

Nach der in Beispiel 23 angegebenen Arbeitsweise werden 39,85 g (0,1 Mol) (4R,5R)-1,3-Dibenzyl-2-oxo-5-acetylmercaptomethyl-imidazolin-4-carbonsäure mit 4,0 g (0,1 Mol) Natriumhydroxid in 100 ml N-Methylpyrrolidon umgesetzt.

Man erhält 22,9 g (3aS,6aR)-1,3-Dibenzyl-tetrahydrothieno[3,4-d]imidazol-2(3H),4-dion mit einem Schmelzpunkt von 119 °C.

$[\alpha]_D^{20}$ = +90,8°, c = 1 ($CHCl_3$)

Beispiel 26

Eine Lösung von 53,47 g (0,15 Mol) (4R,5S)-1,3-Dibenzyl-2-oxo-5-mercaptomethyl-imidazolidin-4-carbonsäure in 300 ml Pyridin wird bei Raumtemperatur mit 1,43 g (7,5 mMol) Toluol-4-sulfonsäure-Monohydrat und 37,14 g (0,18 Mol) N,N'-Dicyclohexylcarbodiimid versetzt und 6 Stunden bei 20 °C gerührt.

Anschließend saugt man ab und engt das Filtrat unter vermindertem Druck ein.

Den Rückstand löst man in 400 ml Toluol, wäscht zweimal mit je 50 ml 1 N Salzsäure, engt ein und kristallisiert aus 2-Propanol um.

Es werden 40,6 g (3aS,6aR)-1,3-Dibenzyltetrahydrothieno[3,4-d]imidazol-2(3H),4-dion mit einem Schmelzpunkt von 119-120 °C erhalten.

$[\alpha]_D^{20}$ = +91,3 °, c = 1 ($CHCl_3$)

Beispiel 27

Eine Lösung von 5,35 g (15 mMol) (4R,5R)-1,3-Dibenzyl-2-oxo-5-mercaptomethyl-imidazolidin-4-carbonsäure in einem Gemisch aus 50 ml Dichlormethan und 1,42 g (18 mMol) Pyridin wird bei 5 °C mit 1,70 g (18 mMol) Methylchlorformiat versetzt und 36 Stunden unter Rückfluß gekocht.

Anschließend wird auf Raumtemperatur abgekühlt, zweimal mit je 25 ml 1 N Salzsäure gewaschen, über Natriumsulfat getrocknet und unter vermindertem Druck eingeengt.

Der Rückstand wird an Kieselgel mit Toluol/Ethylacetat (9:1, v/v) chromatographiert.

Man erhält 3,1 g (3aS,6aR)-1,3-Dibenzyltetrahydrothieno[3,4-d]imidazol-2(3H),4-dion mit einem Schmelzpunkt von 119 °C.

$[\alpha]_D^{20}$ = +90,9°, c = 1 ($CHCl_3$)

Beispiel 28

Eine Lösung von 9,92 g (28 mMol) (7S,7aR)-3-Phenyl-5(6H)-oxo-6-benzyl-7,7a-dihydro-1H,3H-imidazo-[1,5-c]thiazol-7-carbonsäure in 150 ml wasserfreier Essigsäure wird bei 80 °C portionsweise mit 7,32 g (112 mMol) Zinkpulver versetzt, weitere 4 Stunden bei 80 °C gerührt, filtriert und unter vermindertem Druck eingeengt.

Den Rückstand löst man in 300 ml Toluol, wäscht mit 150 ml Wasser und 75 ml gesättigter Kochsalzlösung und engt unter vermindertem Druck ein. Das rohe Thiolacton wird aus Ethylacetat umkristallisiert.

Man erhält 8,0 g (3aS,6aR)-1,3-Dibenzyl-tetrahydrothieno[3,4-d]imidazol-2(3H),4-dion mit einem Schmelzpunkt von 119-120 °C.

$[\alpha]_D^{20}$ = +91,2°, c = 1 ($CHCl_3$)

Beispiel 29

Eine Lösung von 99,24 g (0,28 Mol) (7R,7aR)- und (7S,7aR)-3-Phenyl-5(6H)-oxo-6-benzyl-7,7a-dihydro-1H, 3H-imidazo[1,5-c]thiazol-7-carbonsäure in 1500 ml wasserfreier Essigsäure wird bei 80 °C portionsweise mit 73,22 g (1,12 Mol) Zinkpulver versetzt, weitere 4 Stunden bei 80 °C gerührt, filtriert und unter vermindertem Druck eingeengt.

Der Rückstand wird in 1000 ml Dimethylformamid gelöst, die Lösung mit 38,1 g (0,28 Mol) Natriumacetat-Trihydrat versetzt und 60 Minuten bei 125 °C gerührt.

Anschließend kühlt man auf Raumtemperatur ab, rührt in 1500 ml Wasser ein, extrahiert viermal mit je 200 ml Toluol, wäscht die vereinigten Extrakte dreimal mit je 150 ml Wasser und einmal mit gesättigter Kochsalzlösung und engt unter vermindertem Druck ein.

Den Rückstand kristallisiert man aus Ethylacetat um und erhält 75,8 g (3aS,6aR)-1,3-Dibenzyl-tetrahydro-thieno[3,4-d]imidazol-2(3H),4-dion mit einem Schmelzpunkt von 118-119 °C.

$[\alpha]_D^{20}$ = +90,6°, c = 1 ($CHCl_3$)

Beispiel 30

Man suspendiert 10,06 g (30 mMol) (7S,7aR)-3-Phenyl-6-benzyl-7-cyano-7,7a-dihydro-1H,3H-imidazo-[1,5-c]thiazol-5(6H)-on in 150 ml gesättigter methanolischer Salzsäure und rührt das Gemisch 5 Stunden bei 60 °C.

Anschließend wird abgekühlt, mit 500 ml Eiswasser zersetzt und viermal mit je 150 ml Ethylacetat extrahiert.

Die vereinigten organischen Phasen werden mit insgesamt 300 ml Wasser gewaschen, über Natriumsulfat getrocknet und unter vermindertem Druck eingeengt. Beim Verreiben mit Diethylether kristallisiert das Produkt.

Man erhält 10,2 g (7S,7aR)-3-Phenyl-5(6H)-oxo-6-benzyl-7,7a-dihydro-1H,3H-imidazo[1,5-c]-thiazol-7-carbonsäuremethylester mit einem Schmelzpunkt von 155-156 °C.

$$[\alpha]_{365}^{20} = -752°,$$

c = 1 (Methanol)

Beispiel 31

Aus 10,06 g (30 mMol) (7R,7aR)-3-Phenyl-6-benzyl-7-cyano-7,7a-dihydro-1H,3H-imidazo[1,5-c]thiazol-5-(6H)-on werden nach der in Beispiel 15 angegebenen Arbeitsweise 10,1 g (7R,7aR)-3-Phenyl-5(6H)-oxo-6-benzyl-7,7a-dihydro-1H,3H-imidazo[1,5-c]-thiazol-7-carbonsäuremethylester als farbloses Öl erhalten;

$$[\alpha]_{365}^{20} = -862°,$$

c = 1 (Methanol).

Beispiel 32

Eine Lösung von 11,05 g (30 mMol) (7S,7aR)-3-Phenyl-5(6H)-oxo-6-benzyl-7,7a-dihydro-1H,3H-imidazo-[1,5-c]thiazol-7-carbonsäuremethylesterin 150 ml wasserfreier Essigsäure wird bei 80 °C portionsweise mit 7,85 g (120 mMol) Zinkpulver versetzt, weitere 4 Stunden bei 80 °C gerührt, filtriert und unter vermindertem Druck eingeengt.

Den Rückstand löst man in 300 ml Toluol, wäscht mit 150 ml Wasser und 75 ml gesättigter Kochsalzlösung und engt unter vermindertem Druck ein.

Das rohe Thiolacton wird aus Ethylacetat umkristallisiert. Man erhält 8,4 g (3aS,6aR)-1,3-Dibenzyl-tetrahydro-thieno[3,4-d]imidazol-2(3H),4-dion mit einem Schmelzpunkt von 120 °C.
$[\alpha]_D^{20} = +91,1°$, c = 1 ($CHCl_3$)

Beispiel 33

Eine Lösung von 11,05 g (30 mMol) (7R,7aR)-3-Phenyl-5(6H)-oxo-6-benzyl-7,7a-dihydro-1H,3H-imidazo-[1,5-c]thiazol-7-carbonsäuremethylesterin 150 ml wasserfreier Essigsäure wird bei 80 °C portionsweise mit 7,85 g (120 mMol) Zinkpulver versetzt, weitere 4 Stunden bei 80 °C gerührt, filtriert und unter vermindertem Druck eingeengt.

Der Rückstand wird in 110 ml Dimethylformamid gelöst, die Lösung mit 4,08 g (30 mMol) Natriumacetet-Trihydrat versetzt und 60 Minuten bei 110 °C gerührt.

Anschließend kühlt man auf Raumtemperatur ab, rührt in 200 ml Wasser ein, extrahiert viermal mit je 100 ml Toluol, wäscht die vereinigten Extrakte dreimal mit je 50 ml Wasser und einmal mit 50 ml gesättigter Kochsalzlösung und engt unter vermindertem Druck ein.

Den Rückstand kristallisiert man aus Ethylacetat um und erhält 7,5 g (3aS,6aR)-1,3-Dibenzyl-tetrahydrothieno[3,4-d]imidazol-2(3H),4-dion mit einem Schmelzpunkt von 119 °C.
$[\alpha]_D^{20} = + 90,6°$, c = 1 ($CHCl_3$)

Beispiel 34

Eine aus 3,68 g (30 mMol) 1-Chlor-4-methoxybutan und 0,97 g (40 mMol) Magnesium in 15 ml Tetrahydrofuran bereitete Grignardreagenzlösung tropft man bei 40 °C unter Stickstoff zu einer Lösung von 6,71 g (20 mMol) (7R,7aR)-3-Phenyl-6-benzyl-7-cyano-7,7a-dihydro-1H,3H-imidazo[1,5-c]thiazol-5(6H)-on in 10 ml Tetrahydrofuran.

Man rührt 30 Minuten bei 40 °C, zersetzt mit 50 ml Eiswasser und stellt mit 1N Salzsäure pH 4 ein. Anschließend wird dreimal mit je 50 ml Ether extrahiert, über Natriumsulfat getrocknet und unter vermindertem Druck eingeengt. Das Rohprodukt wird an Kieselgel mit Diethylether chromatographiert.

Man erhält 6,6 g (7R,7aR)-3-Phenyl-6-benzyl-7-(5-methoxypentanoyl)-7,7a-dihydro-1H,3H-imidazo[1,5-c]thiazol-5(6H)-on als gelbes Öl.

$$[\alpha]^{20}_{436} = -480°,$$

c = 1 (Methanol)

Beispiel 35

Nach der in Beispiel 19 angegebenen Arbeitsweise werden 3,86 g (30 mMol) 1-Chlor-4-methoxybutan, 0,97 g (40 mMol) Magnesium und 6,71 g (20 mMol) (7S,7aR)-3-Phenyl-6-benzyl-7-cyano-7,7a-dihydro-1H,3H-imidazo[1,5-c]thiazol-5(6H)-on in 25 ml Tetrahydrofuran miteinander umgesetzt.

Man erhält 6,3 g (7R,7aR)-3-Phenyl-6-benzyl-7-(5-methoxypentanoyl)-7,7a-dihydro-1H,3H-imidazo[1,5-c]thiazol-5(6H)-on als gelbes Öl.

$$[\alpha]^{20}_{436} = -475°,$$

c = 1 (Methanol)

Beispiel 36

Eine Lösung von 11,46 g (27 mMol) (7R,7aR)-3-Phenyl-6-benzyl-7-(5-methoxypentanoyl)-7,7a-dihydro-1H,3H-imidazo[1,5-c]thiazol-5(6H)-on in 200 ml wasserfreier Essigsäure wird bei 80 °C portionsweise mit 7,06 g (108 mMol) Zinkpulver versetzt, weitere 5 Stunden bei 80 °C gerührt, filtriert und unter vermindertem Druck eingeengt.

Den Rückstand nimmt man in 150 ml Toluol auf, wäscht dreimal mit je 100 ml Wasser und engt erneut unter vermindertem Druck ein. Als Rückstand wird (4R,5R)-1,3-Dibenzyl-2-oxo-4-(5-methoxypentanoyl)-5-mercaptomethyl-imidazolidin erhalten;

$$[\alpha]^{20}_{365} = -52,6\ °,$$

c = 1 (Methanol).

Man löst das schwach gelbe Öl bei Raumtemperatur in 3,66 g (61 mMol) wasserfreier Essigsäure, tropft unter Eiskühlung 5,19 g (61 mMol) Piperidin zu und erhitzt die Schmelze unter Rühren 90 Minuten auf 100 °C.

Anschließend kühlt man auf 20 °C ab, versetzt mit 150 ml Wasser und extrahiert dreimal mit je 100 ml tert.-Butylmethylether. Die vereinigten organischen Phasen werden mit 1 N Salzsäure und Wasser gewaschen, über Natriumsulfat getrocknet und unter vermindertem Druck eingeengt.

Danach wird die Lösung des öligen Rückstandes in 50 ml Essigsäure 2 Stunden auf 100 °C erhitzt, abgekühlt und unter vermindertem Druck eingeengt.

Den Rückstand nimmt man in 100 ml tert.-Butylmethylether auf, wäscht einmal mit 50 ml 2 N Natronlauge, danach zweimal mit je 50 ml Wasser, trocknet über Natriumsulfat, engt ein und chromatographiert das Rohprodukt an Kieselgel (Diethylether).

Man erhält 8,1 g (3aS,6aR)-1,3-Dibenzyl-4-(4-methoxybutyliden)-tetrahydro-thieno[3,4-d]imidazol-2(3H)-on als Öl.

$[\alpha]^{25}_{D} = +236°$, c = 1 (Benzol)

Beispiel 37

Zu einer unter Stickstoff gerührten Suspension von 19,95 g (45 mMol) (4-Carboxybutyl)-triphenylphosphoniumbromid in 120 ml Tetrahydrofuran tropft man bei 20 °C 57,5 ml (92 mMol) einer 1,6 molaren

Lösung von n-Butyl-lithium in n-Hexan.

Man rührt das Gemisch 40 Minuten bei Raumtemperatur, kühlt auf 0 °C ab, trägt 7,36 g (55 mMol) Lithiumiodid ein und tropft innert 20 Minuten eine Lösung von 13,42 g (40 mMol) eines Gemisches aus (7R,7aR)- und (7S,7aR)-3-Phenyl-6-benzyl-7-cyano-7,7a-dihydro-1H, 3H-imidazo[1,5-c]thiazol-5(6H)-on in 75 ml THF zu.

Anschließend rührt man das Gemisch eine Stunde bei Raumtemperatur, danach 2 Stunden bei 50 °C, kühlt auf 0 °C ab und zersetzt mit einem Gemisch aus 30 ml Eiswasser und 25 ml konz. Salzsäure.

Man rührt 30 Minuten bei Raumtemperatur und destilliert den größten Teil des Lösungsmittels unter vermindertem Druck ab. Der Rückstand wird in 150 ml Ethylacetat aufgenommen.
Nach Phasentrennung wird die organische Lösung zweimal mit je 50 ml Wasser gewaschen, über Natriumsulfat getrocknet und unter vermindertem Druck eingeengt.

Der Rückstand wird in 150 ml wasserfreier Essigsäure gelöst und bei 80 °C portionsweise mit 7,85 g (120 mMol) Zinkpulver versetzt. Man rührt das Gemisch 4 Stunden bei 90 °C, filtriert und engt unter vermindertem Druck ein.

Den öligen Rückstand nimmt man in 100 ml Toluol auf, wäscht dreimal mit je 50 ml Wasser und engt erneut unter vermindertem Druck ein. Als Rückstand wird (4R,5R)-1,3-Dibenzyl-2-oxo-4-(5-carboxypentanoyl)-5-mercaptomethyl-imidazolidin erhalten.

Zur Lösung des Rückstandes in 7,21 g (120 mMol) Essigsäure tropft man bei 0 °C 11,92 g (140 mMol) Piperidin und erhitzt das Gemisch 2 Stunden auf 100 °C.

Anschließend kühlt man auf 25 °C ab, versetzt mit 150 ml Wasser, stellt mit 2 N Salzsäure pH 3 ein und extrahiert dreimal mit je 100 ml Ethylacetat. Die vereinigten organischen Lösungen werden mit 1 N Salzsäure und Wasser gewaschen, über Natriumsulfat getrocknet und unter vermindertem Druck eingeengt. Als Rückstand wird (3aS,4RS,6aR)-1,3-Dibenzyl-4-hydroxy-4-(4-carboxybutyl)-tetrahydro-thieno[3,4-d]-imidazol-2(3H)-on erhalten.

Danach wird die Lösung des öligen Rückstandes in 75 ml Essigsäure 1 Stunde auf 100 °C erhitzt, auf Raumtemperatur abgekühlt und unter vermindertem Druck eingeengt.

Den Rückstand nimmt man in 150 ml Ethylacetat auf, wäscht zweimal mit je 50 ml Wasser, trocknet über Natriumsulfat, engt ein und chromatographiert das Rohprodukt an Kieselgel (Toluol/Ethylacetat 3 : 1, v/v).

Man erhält 10,1 g (3aS,6aR)-1,3-Dibenzyl-4-(4-carboxybutyliden)-tetrahydrothieno[3,4-d]imidazol-2(3H)-on mit einem Schmelzpunkt von 79-82 °C.
$[\alpha]_D^{22} = +215^\circ$, c = 1 (Methanol)

Beispiel 38

Zu einer unter Stickstoff gerührten Suspension von 5,49 g (226 mMol) Magnesiumspänen in einem Gemisch aus 35 ml Diethylether und 35 ml Toluol tropft man bei 23 bis 27 °C innert 15 Minuten eine Lösung von 12,40 g (66 mMol) 1,2-Dibromethan in 18 ml Diethylether, rührt 45 Minuten bei Raumtemperatur und tropft anschließend eine Lösung von 8,38 g (66 mMol) 1,4-Dichlorbutan in einem Gemisch aus 18 ml Diethylether und 38 ml Toluol innert 25 Minuten ohne Kühlung zu.

Man rührt weitere 90 Minuten, kühlt auf -30 °C ab und tropft bei dieser Temperatur eine Lösung von 7,38 g (22 mMol) (7RS,7aR)-3-Phenyl-6-benzyl-7-cyano-7,7a-dihydro-1H,3H-imidazo[1,5-c]-thiazol-5(6H)-on in 150 ml Toluol innert 30 Minuten zu.

Anschließend wird während 60 Minuten Kohlendioxid eingeleitet, wobei man die Temperatur auf 0 °C ansteigen läßt. Danach wird unter vermindertem Druck eingeengt und der Rückstand zwischen 150 ml 1 N Salzsäure und 200 ml Ethylacetat verteilt. Die organische Phase wird abgetrennt, über Natriumsulfat getrocknet und unter vermindertem Druck eingeengt.

Man erhält 6,05 g (7RS,7aR)-3-Phenyl-6-benzyl-7-(5-carboxypentanoyl)-7,7a-dihydro-1H,3H-imidazo[1,5-c]-5-(6H)-on als in Methanol linksdrehendes Öl.

Der Rückstand wird in 100 ml wasserfreier Essigsäure gelöst und bei 80 °C portionsweise mit 5,88 g (90 mMol) Zinkpulver versetzt. Man rührt das Gemisch 4 Stunden bei 90 °C, filtriert und engt unter vermindertem Druck ein.

Zur Lösung des Rückstandes in 5,40 g (90 mMol) Essigsäure tropft man bei 0 °C 8,52 g (100 mMol) Piperidin und erhitzt das Gemisch 2 Stunden auf 100 °C.

Anschließend kühlt man auf 25 °C ab, versetzt mit 100 ml Wasser, stellt mit 2 N Salzsäure pH 3 ein und extrahiert dreimal mit je 50 ml Ethylacetat.

Die vereinigten organischen Lösungen werden mit 1 N Salzsäure und Wasser gewaschen, über Natriumsulfat getrocknet und unter vermindertem Druck eingeengt.

Als Rückstand wird (3aS,4RS,6aR)-1,3-Dibenzyl-4-hydroxy-4-(4-carboxybutyl)-tetrahydrothieno[3,4-d]-imidazol-2(3H)-on als in Methanol rechtsdrehendes Öl erhalten.

Danach wird die Lösung des Rückstandes in 50 ml Essigsäure 1 Stunde auf 100 °C erhitzt und unter vermindertem Druck eingeengt. Den Rückstand nimmt man in 100 ml Ethylacetat auf, wäscht zweimal mit je 50 ml Wasser, trocknet über Natriumsulfat, engt ein und chromatographiert das Rohprodukt an Kieselgel (Toluol/Ethylacetat 3:1, v/v).

Man erhält 4,2 g (3aS,6aR)-1,3-Dibenzyl-4-(4-carboxybutyliden)-tetrahydrothieno[3,4-d]imidazol-2(3H)-on mit einem Schmelzpunkt von 80-82 °C.

$[\alpha]_D^{22}$ = +214 °, c = 1 (Methanol)

Beispiel 39

Zu einer unter Stickstoff gerührten Suspension von 7,53 g (310 mMol) Magnesiumspänen in 50 ml Diethylether tropft man innert 30 Minuten eine Lösung von 19,43 g (90 mMol) 1,4-Dibrombutan in 240 ml Diethylether, kocht 60 Minuten unter Rückfluß und tropft anschließend bei 30 °C eine Lösung von 10,06 g (30 mMol) (7RS,7aR)-3-Phenyl-6-benzyl-7-cyano-7,7a-dihydro-1H,3H-imidazo[1,5-c]thiazol-5(6H)-on in 300 ml Diethylether innert 10 Minuten zu.

Danach kühlt man das Reaktionsgemisch auf -30 °C ab und leitet während 60 Minuten Kohlendioxid ein, wobei man die Temperatur auf 0 °C ansteigen läßt.

Nach Abdestillieren des Lösungsmittels wird der Rückstand zwischen 200 ml 1 N Salzsäure und 200 ml Ethylacetat verteilt. Die organische Phase wird abgetrennt, über Natriumsulfat getrocknet und unter vermindertem Druck eingeengt. Man erhält 7,2 g (7RS,7aR)-3-Phenyl-6-benzyl-7-(5-carboxypentanoyl)-7,7a-dihydro-1H,3H-imidazo[1,5-c]thiazol-5(6H)-on als in Methanol linksdrehendes Öl.

Der Rückstand wird in 150 ml wasserfreier Essigsäure gelöst und bei 80 °C portionsweise mit 6,54 g (100 mMol) Zinkpulver versetzt. Man rührt das Gemisch 4 Stunden bei 90 °C, filtriert und destilliert 140 ml Essigsäure ab.

Nach Zugabe von 17,03 g (200 mMol) Piperidin bei 0 °C` wird das Reaktionsgemisch 2 Stunden auf 100 °C erhitzt, danach mit 150 ml Essigsäure versetzt, weitere 60 Minuten bei 100 °C gerührt und unter vermindertem Druck eingeengt.

Den Rückstand nimmt man in 150 ml Ethylacetat auf, wäscht mit 1 N Salzsäure und Wasser, trocknet über Natriumsulfat und engt unter vermindertem Druck ein.

Als Rückstand wird (3aS,6aR)-1,3-Dibenzyl-4-(4-carboxybutyliden)-tetrahydrothieno[3,4-d]imidazol-2-(3H)-on erhalten.

Das Rohprodukt löst man in 40 ml Methanol, versetzt mit 30 ml gesättigter methanolischer Salzsäure und rührt die Lösung 2 Stunden bei Raumtemperatur. Danach wird unter vermindertem Druck eingeengt und der ölige Rückstand in 100 ml Toluol aufgenommen.

Man wäscht die Lösung zweimal mit je 50 ml Wasser, destilliert das Lösungsmittel ab und chromatographiert das Rohprodukt an Kieselgel (Toluol/Ethylacetat 9:1, v/v).

Man erhält 6,4 g (3aS,6aR)-1,3-Dibenzyl-4-(4-methoxycarbonylbutyliden)-tetrahydrothieno[3,4-d]-imidazol-2(3H)-on als Öl.

$[\alpha]_D^{25}$ = + 227°, c = 1 (Benzol)

Beispiel 40

Nach der in Beispiel 39 angegebenen Arbeitsweise werden 19,43 g (90 mMol) 1,4-Dibrombutan, 7,53 g (310 mMol) Magnesium und 10,06 g (30 mMol) (7R,7aR)-3-Phenyl-6-benzyl-7-cyano-7,7a-dihydro-1H,3H-imidazo[1,5-c]thiazol-5(6H)-on in 290 ml Diethylether umgesetzt.

Man erhält 6,8 g (3aS,6aR)-1,3-Dibenzyl-4-(4-methoxycarbonylbutyliden)-tetrahydrothieno[3,4-d]-imidazol-2(3H)-on als Öl.

$[\alpha]_D^{25}$ = + 225°, c = 1 (Benzol)

Beispiel 41

Nach der in Beispiel 39 angegebenen Arbeitsweise werden 19,43 g (90 mMol) 1,4-Dibrombutan, 7,53 g (310 mMol) Magnesium und 10,06 g (30 mMol) (7S,7aR)-3-Phenyl-6-benzyl-7-cyano-7,7a-dihydro-1H,3H-imidazo[1,5-c]thiazol-5(6H)-on in 290 ml Diethylether umgesetzt.

Man erhält 5,2 g (3aS,6aR)-1,3-Dibenzyl-4-(4-methoxycarbonylbutyliden)-tetrahydrothieno[3,4-d]-imidazol-2(3H)-on als Öl.

$[\alpha]_D^{25}$ = + 223°, c = 1 (Benzol)

Beispiel 42

Zu einer Lösung von 33,54 g (0,1 Mol) eines Gemisches aus (7R,7aR)- und (7S,7aR)-3-Phenyl-6-benzyl-7-cyano-7,7a-dihydro-1H,3H-imidazo[1,5-c]thiazol-5(6H)-on in 250 ml Toluol tropft man unter Stickstoff bei -70 °C 200 ml einer 1 M Lösung von Diisobutylaluminiumhydrid in Toluol, erwärmt innert 3 Stunden auf 0 °C und hält weitere 2 Stunden bei dieser Temperatur. Anschließend wird mit 5 ml Methanol zersetzt und in 250 ml gesättigte Ammoniumchloridlösung eingerührt. Man trennt die Phasen und schüttelt die organische Phase anschließend 20 Minuten mit 100 ml 10 %iger Schwefelsäure.

Man trennt die Phasen, wäscht zweimal mit je 100 ml Wasser, trocknet über Natriumsulfat und engt unter vermindertem Druck ein.

Man erhält 29,4 g eines Gemisches aus (7R,7aR)- und (7S,7aR)-3-Phenyl-5(6H)-oxo-6-benzyl-7,7a-dihydro-1H, 3H-imidazo[1,5-c]thiazol-7-carbaldehyd.

Beispiel 43

In eine unter Stickstoff gerührte Suspension von 19,95 g (45 mMol) (4-Carboxybutyl)-triphenylphosphoniumbromid in 150 ml Tetrahydrofuran tropft man bei 20 °C 57,5 ml (92 mMol) einer 1,6 M Lösung von n-Butyllithium in n-Hexan.

Man rührt das Reaktionsgemisch 60 Minuten bei 20 °C, kühlt auf 0 °C ab und tropft innert 15 Minuten eine Lösung von 13,54 g (40 mMol) eines Gemisches aus (7R,7aR)- und (7S,7aR)-2-Oxo-3-phenyl-6-benzyl-7,7a-dihydro-1H, 3H,6H-imidazo[1,5-c]thiazol-7-carbaldehyd in 50 ml Tetrahydrofuran zu. Nach weiteren 60 Minuten wird das Reaktionsgemisch in 200 ml Eiswasser eingerührt und mit konz. Salzsäure auf pH 2 gebracht. Man trennt die Phasen und extrahiert die wässrige Phase zweimal mit je 50 ml Ethylacetat. Die vereinigten organischen Phasen werden mit Wasser sowie gesättigter Kochsalzlösung gewaschen, über Natriumsulfat getrocknet und unter vermindertem Druck eingeengt. Das Rohprodukt wird anschließend mit Ethylacetat an Kieselgel chromatographiert.

Man erhält 13,9 g eines Gemisches aus (7R,7aR)- und (7S,7aR)-3-Phenyl-6-benzyl-(5-carboxy-pent-1-enyl)-7,7a-dihydro-1H,3H-imidazo[1,5-c]thiazol-5(6H)-on als Öl.

Beispiel 44

Eine Lösung von 12,68 g (30 mMol) eines Gemisches aus (7R,7aR)- und (7S,7aR)-3-Phenyl-6-benzyl-(5-carboxypent-1-enyl)-7,7a-dihydro-1H,3H-imidazo[1,5-c]thiazol-5(6H)-on in 150 ml wasserfreier Essigsäure wird bei 85 °C portionsweise mit 7,85 g (120 mMol) Zinkpulver versetzt, weitere 4 Stunden bei 85 °C gerührt, filtriert und unter vermindertem Druck eingeengt. Der Rückstand wird an Kieselgel chromatographiert (Dichlormethan/Methanol 9 : 1, v/v).

Man erhält 4,8 g (3aS,4S,6aR)-1,3-Dibenzyl-4-(4-carboxybutyl)-tetrahydro-thieno[3,4-d]imidazol-2(3H)-on mit einem Schmelzpunkt von 88-89 °C.
$[\alpha]_D^{22}$ = -24,3°, c = 1 (Methanol)

Beispiel 45

8,77 g (20 mMol) eines nach der in den Beispielen 38-41 beschriebenen Arbeitsweise erhaltenen Gemisches aus (7S, 7aR)- und (7R,7aR)-3-Phenyl-6-benzyl-7-(5-carboxypentanoyl)-7,7a-dihydro-1H,3H-imidazo[1,5-c]thiazol-5(6H)-on werden in 60 ml Methanol gelöst und mit 40 ml gesättigter methanolischer Salzsäure versetzt.

Man rührt 2 Stunden bei Raumtemperatur und engt unter vermindertem Druck ein. Den Rückstand löst man in 100 ml Toluol, wäscht zweimal mit je 50 ml Wasser, destilliert das Lösungsmittel ab und chromatographiert das Rohprodukt an Kieselgel (Ethylacetat).

Man erhält 3,3 g (3aS,6aR)-3-Benzyl-4-(4-methoxycarbonylbutyliden)-tetrahydrothieno[3,4-d]imidazol-2-(3H)-on als Öl.
$[\alpha]_D^{25}$ = + 215°, c = 1 (Methanol)

**Patentansprüche**

**1.** Verfahren zur Herstellung von D-(+)-Biotin aus L-Cystein oder L-Cystin oder L-Serin, dadurch gekennzeichnet, daß die Synthese über ein aus der Verbindung der Formel I

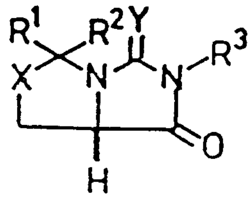

I

hergestelltes Zwischenprodukt der Formel XI geführt wird,

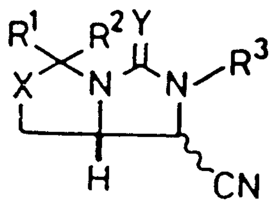

XI

worin jeweils

| | |
|---|---|
| $R^1$ und $R^2$ | jeweils unabhängig voneinander H, unsubstituiertes oder substituiertes niederes Alkyl, Cycloalkyl, Aryl, Aralkyl oder Heteroaryl oder Zusammengenommen unsubstituiertes oder substituiertes Alkylen oder Heteroalkylen, |
| $R^3$ | H oder eine für ein Stickstoffatom geeignete Schutzgruppe und |
| X und Y | jeweils unabhängig voneinander O oder S bedeuten. |

**2.** Verfahren nach Anspruch 1, dadurch gekennzeichnet, daß man die Verbindung der Formel I mit komplexen Hydriden zu einem Alkohol der Formel IX reduziert,

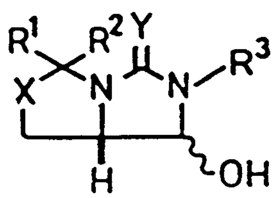

IX

worin $R^1$, $R^2$, $R^3$, X und Y die angegebene Bedeutung besitzen, diesen durch Umsetzung mit einer reaktiven den Rest $R^4$ tragenden Verbindung in einen aktivierten Ester der Formel X überführt,

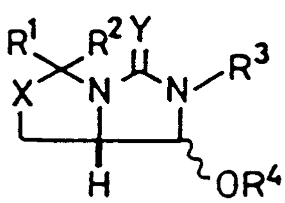

X

worin $R^1$, $R^2$, $R^3$, X und Y die angegebene Bedeutung besitzen und der Rest $R^4$ für eine aktivierende Estergruppe steht, und diesen mit einem Alkali- oder Erdalkalicyanid oder einem Cyanosilan zu einem Nitril der Formel XI umsetzt.

**3.** Verfahren nach Anspruch 2, dadurch gekennzeichnet, daß man den Alkohol der Formel IX mit einer Verbindung der Formel $R^4$-Q umsetzt,

worin

$R^4$ Alkanoyl und
Q Halogen oder Alkanoyloxy

bedeutet.

**4.** Verfahren nach Anspruch 2 oder 3, dadurch gekennzeichnet, daß man den aktivierten Ester der Formel X mit Trimethylsilylcyanid in Gegenwart von Zinn(IV)-chlorid, Titan(IV)-chlorid, Zinn(IV)-triflat oder Zinkbromid umsetzt.

**5.** Verfahren nach Anspruch 1, dadurch gekennzeichnet, daß man das Nitril der Formel XI mit einer Base oder einer Säure zu einem Säurederivat der Formel XII umsetzt,

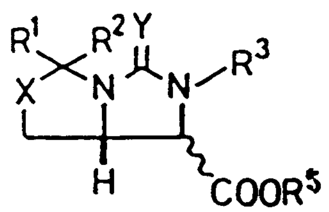

XII

worin $R^1$, $R^2$, $R^3$, X und Y die angegebene Bedeutung besitzen und $R^5$ H oder niederes Alkyl bedeutet, dieses unter Wasserabspaltung zu einem Lacton der Formel XIII cyclisiert,

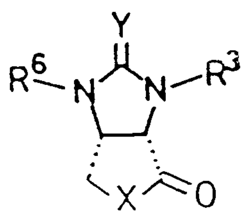

XIII

worin $R^3$, X und Y die angegebene Bedeutung besitzen und $R^6$ H oder $R^1R^2CH$ bedeutet, und dieses nach bekannten Verfahren in D-(+)-Biotin überführt.

**6.** Verfahren nach Anspruch 5, dadurch gekennzeichnet, daß man das Säurederivat der Formel XII mit Zink in Gegenwart einer Säure unter Zusatz eines Acylierungsmittels zu einer Verbindung der Formel XIIa umsetzt,

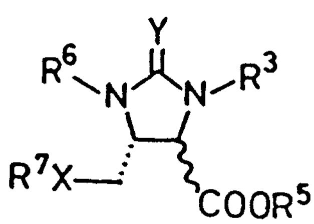

XIIa

worin X, Y, $R^3$ und $R^5$ die angegebene Bedeutung besitzen, und

$R^6$ $CHR^1R^2$ und
$R^7$ Alkanoxyl oder Aroyl

bedeuten, und diese nach Überführung in ein Alkalimetallsalz, worin $R^5$ Natrium oder Kalium bedeutet,

einer thermischen Epimerisierungs- und Cyclisierungsreaktion unter Gewinnung von XIII unterwirft.

**7.** Verfahren nach Anspruch 1, dadurch gekennzeichnet, daß man das Nitril der Formel XI mit einer metallorganischen Verbindung zu einer Oxoverbindung der Formel XIV umsetzt,

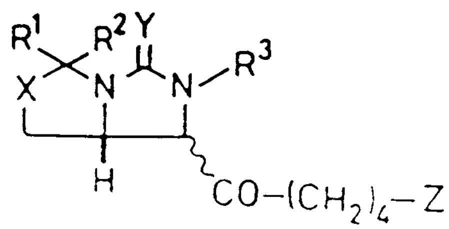

XIV

worin $R^1$, $R^2$, $R^3$, X und Y die angegebene Bedeutung besitzen und Z $OR^5$ oder $COOR^5$ bedeutet, wobei $R^5$ H, niederes Alkyl, Cycloalkyl oder Aryl bedeutet, diese durch Behandeln mit einer Säure und/oder einem Reduktionsmittel zu einem Imidazolidin der Formel XV spaltet,

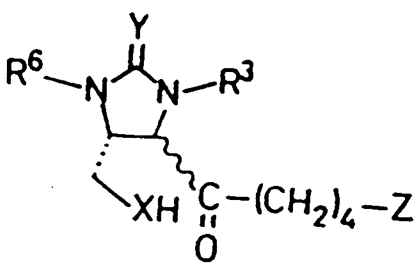

XV

worin $R^3$, $R^6$, X, Y und Z die angegebene Bedeutung besitzen, dieses unter Einwirkung einer Base zu einem Biotinderivat der Formel XVI cyclisiert,

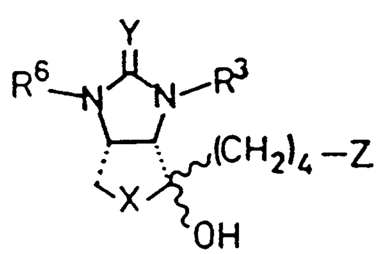

XVI

worin $R^3$, $R^6$, X, Y und Z die angegebene Bedeutung besitzen, das nach bekannten Verfahren in D-(+)-Biotin überführt wird.

**8.** Verfahren nach Anspruch 7, dadurch gekennzeichnet, daß man die Oxoverbindung der Formel XIV mit Zink in Gegenwart einer Säure in eine Verbindung der Formel XV überführt und diese unter basischen Bedingungen zu den Hemiacetalen der Formel XVI cyclisiert.

**9.** Verfahren nach Anspruch 1, dadurch gekennzeichnet, daß man das Nitril der Formel XI mit einem komplexen Hydrid zu einem Aldehyd der Formel XVII umsetzt,

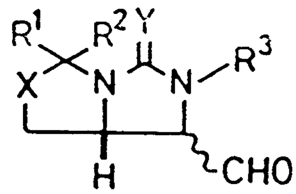

XVII

worin $R^1$, $R^2$, $R^3$, X und Y die angegebene Bedeutung besitzen, diesen mit einer Organophosphorverbindung zu einer ungesättigten Carbonsäure der Formel XVIII kondensiert,

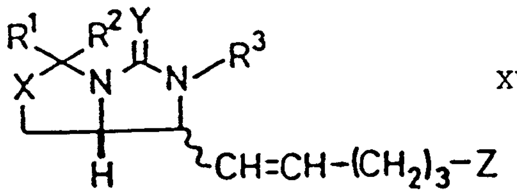

XVIII

worin $R^1$, $R^2$, $R^3$, X, Y und Z die angegebene Bedeutung besitzen, diese durch eine Säure und/oder ein Reduktionsmittel zu einem Biotinderivat der Formel XIX umsetzt,

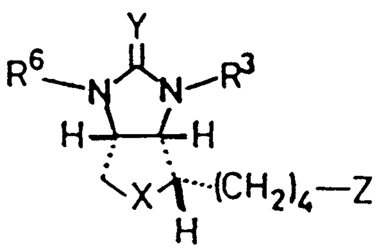

XIX

worin $R^3$, $R^6$, X, Y und Z die angegebene Bedeutung besitzen, und dieses nach bekannten Verfahren in D-(+)-Biotin überführt.

**10.** Verfahren nach Anspruch 9, dadurch gekennzeichnet, daß man das Nitril der Formel XI mit Diisobutylaluminiumhydrid zur Verbindung der Formel XVII reduziert.

**11.** Verfahren nach Anspruch 9 oder 10 , dadurch gekennzeichnet, daß man den Aldehyd der Formel XVII mit einem reaktiven Phosphorylid kondensiert und die erhaltene ungesättigte Carbonsäure der Formel XVIII mit Zink in Gegenwart einer Säure zur Verbindung der Formel XIX umsetzt.

**12.** Verfahren nach Anspruch 1, dadurch gekennzeichnet, daß man das Nitril der Formel XI in eine Oxoverbindung der voranstehend angegebenen Formel XIV überführt, diese unter Säureeinwirkung zu einem Biotinderivat der Formel XX umsetzt,

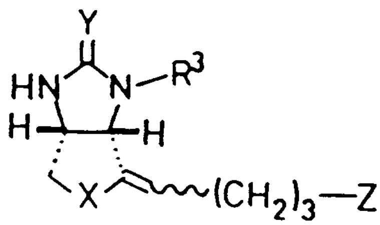

XX

worin $R^3$, X, Y und Z die angegebene Bedeutung besitzen, das nach bekannten Verfahren in D-(+)-Biotin überführt wird.

**13.** Bicyclische Nitrile der Formel XI

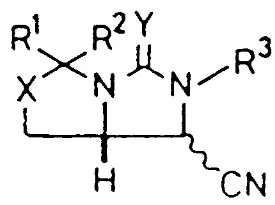

XI,

worin $R^1$, $R^2$, X und Y die in Anspruch 1 angegebene Bedeutung besitzen und $R^3$ eine für ein Stickstoffatom geeignete Schutzgruppe darstellt.

**14.** Bicyclische Nitrile nach Anspruch 13, dadurch gekennzeichnet, daß X Schwefel und Y Sauerstoff bedeuten.

**15.** Das bicyclische Nitril (7RS,7aR)-3-Phenyl-6-benzyl-7-cyano-7,7a-dihydro-1H,3H-imidazo[1,5-c]thiazol-5-(6H)-on der Formel XIa

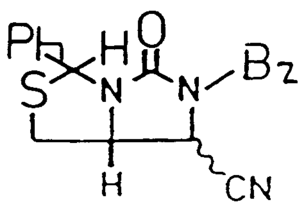

XIa

**16.** Verwendung der bicyclischen Nitrile der Formel XI zur Herstellung von Biotin.

## Claims

**1.** Process for the preparation of D-(+)-biotin from L-cysteine or L-cystine or L-serine, characterised in that the synthesis is carried out via an intermediate product of the formula XI

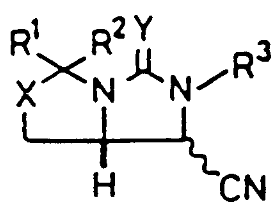

XI

prepared from the compound of the formula I

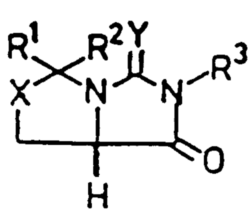

I

wherein, in each case,

| | |
|---|---|
| $R^1$ and $R^2$ | are each independently of one another H, unsubstituted or substituted lower alkyl, cycloalkyl, aryl, aralkyl or heteroaryl or taken together are unsubstituted or substituted alkylene or heteroalkylene, |
| $R^3$ | is H or a protective group which is suitable for a nitrogen atom and |
| X and Y | independently of one another are each O or S. |

2. Process according to Claim 1, characterised in that the compound of the formula I is reduced with complex hydrides to an alcohol of the formula IX

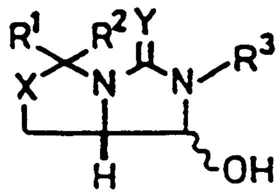

IX

wherein $R^1$, $R^2$, $R^3$, X and Y have the meaning given, this is converted, by reaction with a reactive compound carrying the radical $R^4$ into an activated ester of the formula X

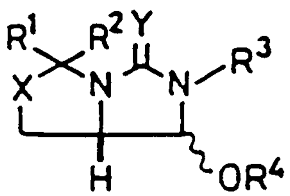

X

wherein $R^1$, $R^2$, $R^3$, X and Y have the meaning given and the radical $R^4$ is an activating ester group, and this is reacted with an alkali metal cyanide or alkaline earth metal cyanide or a cyanosilane to give a nitrile of the formula XI.

3. Process according to Claim 2, characterised in that the alcohol of the formula IX is reacted with a compound of the formula $R^4$-Q, wherein $R^4$ is alkanoyl and Q is halogen or alkanoyloxy.

4. Process according to Claim 2 or 3, characterised in that the activated ester of the formula X is reacted with trimethylsilyl cyanide in the presence of tin(IV) chloride, titanium(IV) chloride, tin(IV) triflate or zinc bromide.

5. Process according to Claim 1, characterised in that the nitrile of the formula XI is reacted with a base or an acid to give an acid derivative of the formula XII

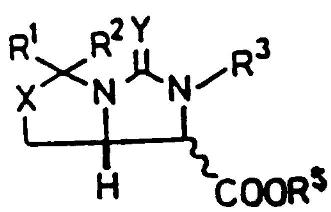

XII

wherein $R^1$, $R^2$, $R^3$, X and Y have the meaning given and $R^5$ is H or lower alkyl, this is cyclised, water being split off, to give a lactone of the formula XIII

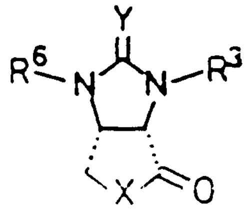

XIII

wherein $R^3$, X and Y have the meaning given and $R^6$ is H or $R^1R^2CH$, and this is converted by known processes into D-(+)-biotin.

**6.** Process according to Claim 5, characterised in that the acid derivative of the formula XII is reacted with zinc in the presence of an acid with addition of an acylating agent to give a compound of the formula XIIa

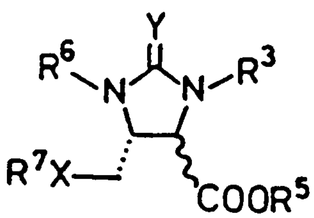

XIIa

wherein X, Y, $R^3$ and $R^5$ have the meaning given, and $R^6$ is $CHR^1R^2$ and $R^7$ is alkanoxyl or aroyl, and after conversion to an alkali metal salt, wherein $R^5$ is sodium or potassium, this is subjected to a thermal epimerisation and cyclisation reaction in which XIII is obtained.

**7.** Process according to Claim 1, characterised in that the nitrile of the formula XI is reacted with an organometallic compound to give an oxo compound of the formula XIV

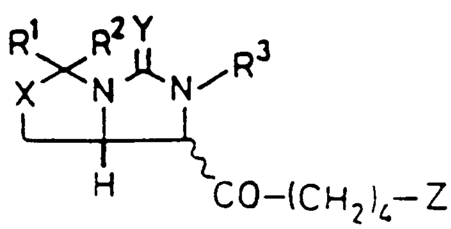

XIV

wherein $R^1$, $R^2$, $R^3$, X and Y have the meaning given and Z is $OR^5$ or $COOR^5$, wherein $R^5$ is H, lower alkyl, cycloalkyl or aryl, this is split by treatment with an acid and/or a reducing agent to give an imidazolidine of the formula XV

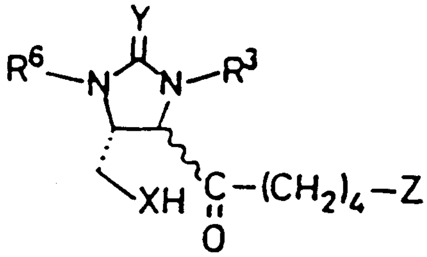

XV

wherein $R^3$, $R^6$, X, Y and Z have the meaning given, this is cyclised, under the action of a base, to give a biotin derivative of the formula XVI.

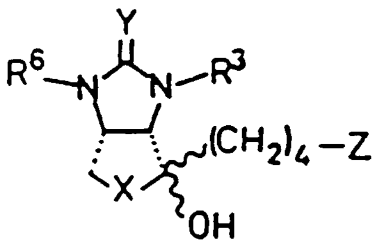

XVI

wherein $R^3$, $R^6$, X, Y and Z have the meaning given, and this is converted into D-(+)-biotin by known processes.

**8.** Process according to Claim 7, characterised in that the oxo compound of the formula XIV is converted with zinc into a compound of the formula XV in the presence of an acid, and this is cyclised under basic conditions to give the hemiacetals of the formula XVI.

**9.** Process according to Claim 1, characterised in that the nitrile of the formula XI is reacted with a complex hydride to give an aldehyde of the formula XVII

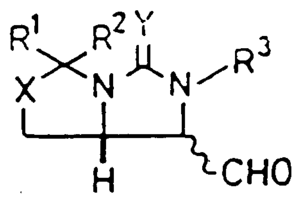

XVII

wherein $R^1$, $R^2$, $R^3$, X and Y have the meaning given, this is condensed with an organophosphorus compound to give an unsaturated carboxylic acid of the formula XVIII

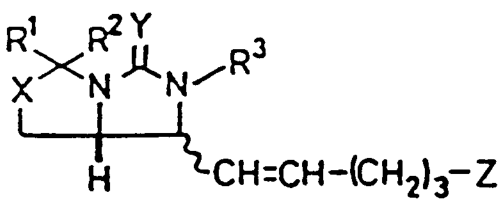

XVIII

wherein $R^1$, $R^2$, $R^3$, X, Y and Z have the meaning given, this is converted by an acid and/or a reducing agent into a biotin derivative of the formula XIX

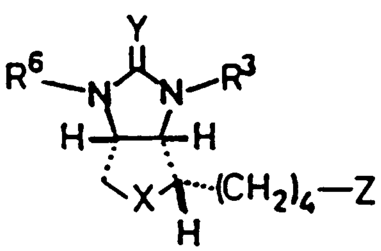

XIX

wherein $R^3$, $R^6$, X, Y and Z have the meaning given, and this is converted into D-(+)-biotin by known processes.

**10.** Process according to Claim 9, characterised in that the nitrile of the formula XI is reduced with diisobutylaluminium hydride to give a compound of the formula XVII.

11. Process according to Claim 9 or 10, characterised in that the aldehyde of the formula XVII is condensed with a reactive phosphorusylide and the unsaturated carboxylic acid of the formula XVIII obtained is reacted with zinc in the presence of an acid to give a compound of the formula XIX.

12. Process according to Claim 1, characterised in that the nitrile of the formula XI is converted into an oxo compound of the abovementioned formula XIV, this is reacted, under the action of an acid, to give a biotin derivative of the formula XX

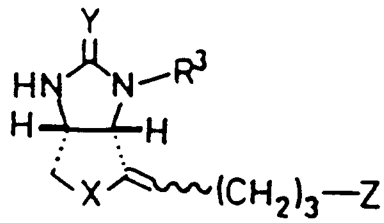

XX

wherein $R^3$, X, Y and Z have the meaning given, and this is converted into D-(+)-biotin by known processes.

13. Bicyclic nitriles of the formula XI

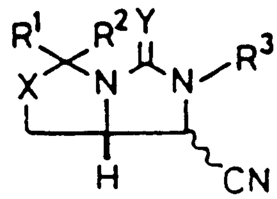

XI,

wherein $R^1$, $R^2$, X and Y have the meaning given in Claim 1 and $R^3$ is a protective group suitable for a nitrogen atom.

14. Bicyclic nitriles according to Claim 13, characterised in that X is sulfur and Y is oxygen.

15. The bicyclic nitrile (7RS,7aR)-3-phenyl-6-benzyl-7-cyano-7,7a-dihydro-1H,3H-imidazo[1,5-c]thiazol-5-(6H)-one of the formula XIa

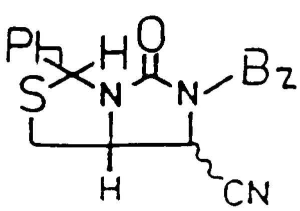

XIa

16. Use of the bicyclic nitriles of the formula XI for the preparation of biotin.

**Revendications**

1. Procédé de préparation de la D-(+)-biotine à partir de la L-cystéine ou de la L-cystine ou de la L-sérine, caractérisé en ce que la synthèse est réalisée en passant par un produit intermédiaire de formule XI

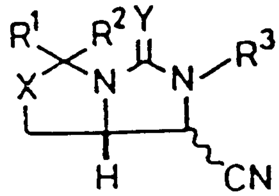

XI

lui-même préparé à partir du composé de formule I

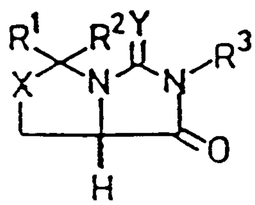

I

les symboles des formules I et XI ayant les significations suivantes :

- $R^1$ et $R^2$ représentent chacun, indépendamment l'un de l'autre, H, un groupe alkyle inférieur, cycloalkyle, aryle, aralkyle ou hétéroaryle non substitué ou substitué ou forment ensemble un groupe alkylène ou hétéroalkylène non substitué ou substitué,
- $R^3$ représente H ou un groupe protecteur approprié pour un atome d'azote, et
- X et Y représentent chacun, indépendamment l'un de l'autre, O ou S.

**2.** Procédé selon la revendication 1, caractérisé en ce que l'on réduit le composé de formule I, à l'aide d'hydrures complexes, en un alcool de formule IX

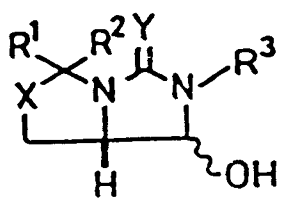

IX

dans laquelle $R^1$, $R^2$, $R^3$, X et Y ont les significations indiquées ci-dessus, qu'on convertit par réaction avec un composé réactif portant le substituant $R^4$, en un ester activé de formule X

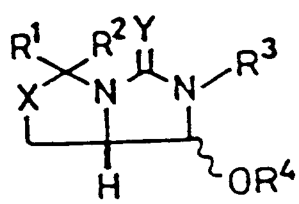

X

dans laquelle $R^1$, $R^2$, $R^3$, X et Y ont les significations indiquées ci-dessus et le substituant $R^4$ est un groupe ester activant, qu'on fait réagir avec un cyanure de métal alcalin ou alcalino-terreux ou un cyanosilane, ce qui donne un nitrile de formule XI.

**3.** Procédé selon la revendication 2, caractérisé en ce que l'on fait réagir l'alcool de formule IX avec un composé de formule $R^4$-Q dans laquelle $R^4$ représente un groupe alcanoyle, et Q représente un halogène ou un groupe alcanoyloxy.

**4.** Procédé selon la revendication 2 ou 3, caractérisé en ce que l'on fait réagir l'ester activé de formule X

avec le cyanure de triméthylsilyle en présence de chlorure d'étain-IV, de chlorure de titane-IV, de triflate d'étain-IV ou de bromure de zinc.

5. Procédé selon la revendication 1, caractérisé en ce que l'on fait réagir le nitrile de formule XI avec une base ou un acide, la réaction donnant un dérivé d'acide de formule XII

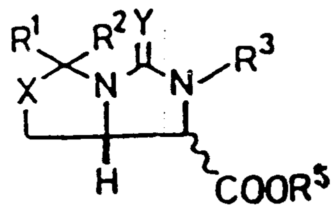

XII

dans laquelle $R^1$, $R^2$, $R^3$, X et Y ont les significations indiquées ci-dessus et $R^5$ représente H ou un groupe alkyle inférieur,
qu'on cyclise, avec séparation d'eau, en une lactone de formule XIII

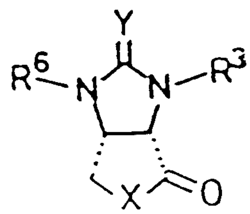

XIII

dans laquelle $R^3$, X et Y ont les significations indiquées ci-dessus et $R^6$ représente H ou $R^1R^2CH$, qu'on convertit par des procédés connus en la D-(+)-biotine.

6. Procédé selon la revendication 5, caractérisé en ce que l'on fait réagir le dérivé d'acide de formule XII avec le zinc en présence d'un acide, avec addition d'un agent acylant, ce qui donne un composé de formule XIIa

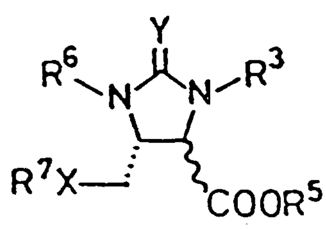

XIIa

dans laquelle X, Y, $R^3$ et $R^5$ ont les significations indiquées ci-dessus, et
$R^6$ représente $CHR^1R^2$, et
$R^7$ représente un groupe alcanoxyle ou aroyle,
qu'on soumet, après conversion en un sel de métal alcalin pour lequel $R^5$ représente le sodium ou le potassium, à une réaction d'épimérisation et de cyclisation à la chaleur donnant le composé XIII.

7. Procédé selon la revendication 1, caractérisé en ce que l'on fait réagir le nitrile de formule XI avec un composé organométallique, la réaction donnant un composé en oxo de formule XIV

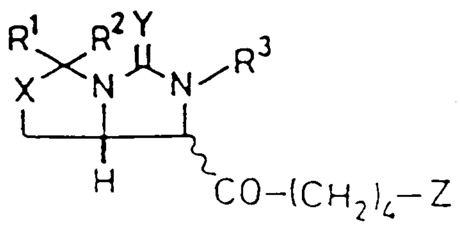

XIV

dans laquelle $R^1$, $R^2$, $R^3$, X et Y ont les significations indiquées ci-dessus et Z représente $OR^5$ ou $COOR^5$, $R^5$ représentant H, un groupe alkyle inférieur, cycloalkyle ou aryle,
qu'on scinde, par traitement à l'aide d'un acide et/ou d'un agent réducteur, en une imidazolidine de formule XV

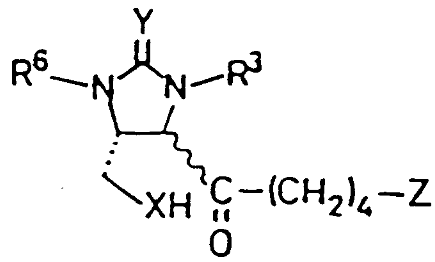

XV

dans laquelle $R^3$, $R^6$, X, Y et Z ont les significations indiquées ci-dessus,
qu'on cyclise sous l'action d'une base en un dérivé de la biotine de formule XVI

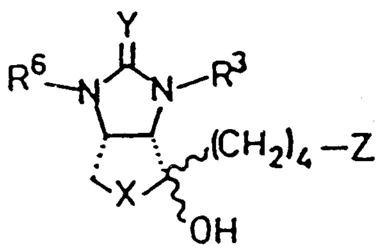

XVI

dans laquelle $R^3$, $R^6$, X, Y et Z ont les significations indiquées ci-dessus,
qu'on convertit par des procédés connus en la D-(+)-biotine.

**8.** Procédé selon la revendication 7, caractérisé en ce que l'on convertit le composé en oxo de formule XIV, à l'aide du zinc en présence d'un acide, en un composé de formule XV qu'on cyclise en milieu basique en les hémi-acétals de formule XVI.

**9.** Procédé selon la revendication 1, caractérisé en ce que l'on fait réagir le nitrile de formule XI avec un hydrure complexe, la réaction donnant un aldéhyde de formule XVII

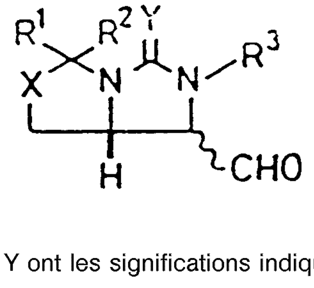

XVII

dans laquelle $R^1$, $R^2$, $R^3$, X et Y ont les significations indiquées ci-dessus,

qu'on condense avec un composé organophosphoré, en un acide carboxylique insaturé de formule XVIII

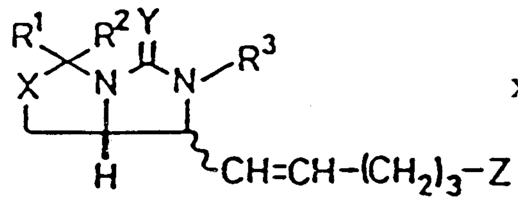

XVIII

dans laquelle $R^1$, $R^2$, $R^3$, X, Y et Z ont les significations indiquées ci-dessus, qu'on convertit à l'aide d'un acide et/ou d'un agent réducteur en un dérivé de la biotine de formule XIX

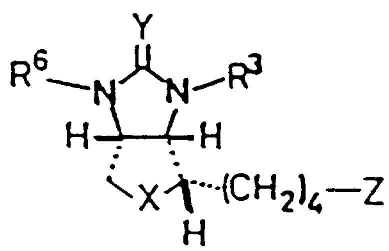

XIX

dans laquelle $R^3$, $R^6$, X, Y et Z ont les significations indiquées ci-dessus, qu'on convertit par des procédés connus en la D-(+)-biotine.

**10.** Procédé selon la revendication 9, caractérisé en ce que l'on convertit le nitrile de formule XI en le composé de formule XVII par réduction à l'aide de l'hydrure de diisobutyl-aluminium.

**11.** Procédé selon la revendication 9 ou 10, caractérisé en ce que l'on condense l'aldéhyde de formule XVII avec un phosphorylure réactif, la réaction donnant un acide carboxylique insaturé de formule XVIII qu'on fait réagir avec le zinc en présence d'un acide, ce qui donne le composé de formule XIX.

**12.** Procédé selon la revendication 1, caractérisé en ce que l'on convertit le nitrile de formule XI en un composé en oxo de formule XIV ci-dessus qu'on convertit sous l'action d'un acide en un dérivé de la biotine de formule XX

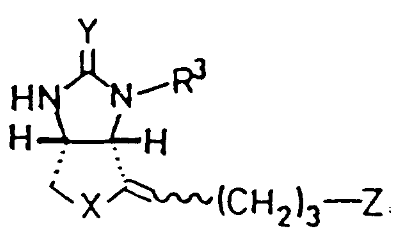

XX

dans laquelle $R^3$, X, Y et Z ont les significations indiquées ci-dessus,
qu'on convertit par des procédés connus en la D-(+)-biotine.

**13.** Nitriles bicycliques de formule XI

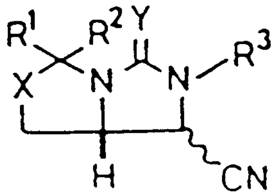

XI,

dans laquelle $R^1$, $R^2$, X et Y ont les significations indiquées ci-dessus dans la revendication 1 et $R^3$ représente un groupe protecteur approprié pour un atome d'azote.

**14.** Nitriles bicycliques selon revendication 13, caractérisés en ce que X représente le soufre et Y l'oxygène.

**15.** Le nitrile bicyclique (7RS,7aR)-3-phényl-6-benzyl-7-cyano-7,7a-dihydro-1H,3H-imidazo[1,5-c]thiazole-5-(6H)-one de formule XIa.

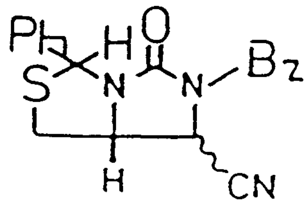

XIa

**16.** Utilisation des nitriles bicycliques de formule XI pour la préparation de la biotine.